# EUROPEAN PATENT SPECIFICATION

(11) **EP 4 034 096 B1**
(45) Date of publication and mention of the grant of the patent: **31.12.2025**
(21) Application number: 20789479.1
(22) Date of filing: 25.09.2020
(51) Int. Cl.: A61K 31/19, A61K 35/74, C12N 5/078

(54) **SHORT-CHAIN FATTY ACID PENTANOATE AS ENHANCER FOR CELLULAR THERAPY AND ANTI-TUMOR THERAPY**
KURZKETTIGES FETTSÄUREPENTANOAT ALS VERSTÄRKER FÜR ZELLTHERAPIE UND ANTITUMORTHERAPIE
PENTANOATE D'ACIDE GRAS À CHAÎNE COURTE EN TANT QU'ACTIVATEUR POUR UNE THÉRAPIE CELLULAIRE ET UNE THÉRAPIE ANTITUMORALE

(30) Priority: 25.09.2019 EP 19199515
(43) Date of publication of application: 03.08.2022
(73) Proprietor: Philipps-Universität Marburg, 35037 Marburg (DE); Julius-Maximilians-Universität Würzburg, 97070 Würzburg (DE)
(72) Inventor: HUDECEK, Michael, 97204 Höchberg (DE); LUU, Maik, 35039 Marburg (DE); VISEKRUNA, Alexander, 35037 Marburg (DE)
(74) Representative: Hoffmann Eitle
(86) International application number: PCT/EP2020/077019
(87) International publication number: WO 2021/058811

(56) References cited:
- WO-A2-2015/006355
- BACHEM A ET AL: "Microbiota-derived butyrate promotes metabolism and memory potential of effector CD8 T cells", vol. 204, no. 1, Supplement, 1 May 2020 (2020-05-01), XP009524221, ISSN: 1550-6606, Retrieved from the Internet <URL:https://www.jimmunol.org/content/204/1_Supplement/72.3>
- PATRICK M SMITH ET AL: "The Microbial Metabolites, Short-Chain Fatty Acids, Regulate Colonic Treg Cell Homeostasis", SCIENCE, vol. 341, no. 6145, 2 August 2013 (2013-08-02), US, pages 569 - 573, XP055247108, ISSN: 0036-8075, DOI: 10.1126/science.1241165
- JI LIANGLIANG ET AL: "Sweet Memories of 8 Empowered by Butyrate", IMMUNITY, CELL PRESS, AMSTERDAM, NL, vol. 51, no. 2, 20 August 2019 (2019-08-20), pages 201 - 203, XP085784544, ISSN: 1074-7613, [retrieved on 20190820], DOI: 10.1016/J.IMMUNI.2019.07.005
- Y KAM ET AL: "238 Effects of short-chain fatty acid on proliferation, metabolic poise, and activation capacity of T cells", CYTOTHERAPY, 1 January 2020 (2020-01-01), XP055752808
- MAIK LUU ET AL: "The short-chain fatty acid pentanoate suppresses autoimmunity by modulating the metabolic-epigenetic crosstalk in lymphocytes", NATURE COMMUNICATIONS, vol. 10, no. 1, 15 February 2019 (2019-02-15), XP055752800, DOI: 10.1038/s41467-019-08711-2
- J PARK ET AL: "Short-chain fatty acids induce both effector and regulatory T cells by suppression of histone deacetylases and regulation of the mTOR-S6K pathway", MUCOSAL IMMUNOLOGY, vol. 8, no. 1, 11 June 2014 (2014-06-11), US, pages 80 - 93, XP055709471, ISSN: 1933-0219, DOI: 10.1038/mi.2014.44
- WEBER ET AL: "Butyrate differentially regulates cytokines and proliferation in porcine peripheral blood mononuclear cells", VETERINARY IMMUNOLOGY AND IMMUNOPATHOLOGY, ELSEVIER BV, AMSTERDAM, NL, vol. 113, no. 1-2, 15 September 2006 (2006-09-15), pages 139 - 147, XP005583040, ISSN: 0165-2427, DOI: 10.1016/J.VETIMM.2006.04.006
- BACHEM ANNABELL ET AL: "Microbiota-Derived Short-Chain Fatty Acids Promote the Memory Potential of Antigen-Activated CD8+ T Cells", IMMUNITY, CELL PRESS, AMSTERDAM, NL, vol. 51, no. 2, 1 July 2019 (2019-07-01), pages 285, XP085784488, ISSN: 1074-7613, [retrieved on 20190701], DOI: 10.1016/J.IMMUNI.2019.06.002
- YUKIHIRO FURUSAWA ET AL: "Commensal microbe-derived butyrate induces the differentiation of colonic regulatory T cells", NATURE, vol. 504, no. 7480, 13 November 2013 (2013-11-13), pages 446 - 450, XP055178312, ISSN: 0028-0836, DOI: 10.1038/nature12721
- SCHRODER CLAUDIA P ET AL: "TRIBUTYRIN ENHANCES THE CYTOTOXIC ACTIVITY OF INTERLEUKIN-2/INTERLEUKIN-12 STIMULATED HUMAN NATURAL KILLER CELLS AGAINST LS 174T COLON CANCER CELLS IN VITRO", CANCER IMMUNOLOGY, IMMUNOTHERAPY, SPRINGER, BERLIN/HEIDELBERG, vol. 50, no. 2, 1 May 2001 (2001-05-01), pages 69 - 76, XP008079171, ISSN: 0340-7004, DOI: 10.1007/S002620100170
- TANOUE TAKESHI ET AL: "A defined commensal consortium elicits CD8 T cells and anti-cancer immunity", NATURE, MACMILLAN JOURNALS LTD, LONDON, vol. 565, no. 7741, 23 January 2019 (2019-01-23), pages 600 - 605, XP036694915, ISSN: 0028-0836, [retrieved on 20190123], DOI: 10.1038/S41586-019-0878-Z
- NICHOLAS ARPAIA ET AL: "Metabolites produced by commensal bacteria promote peripheral regulatory T-cell generation", NATURE, vol. 504, no. 7480, 13 November 2013 (2013-11-13), London, pages 451 - 455, XP055247144, ISSN: 0028-0836, DOI: 10.1038/nature12726
- KOH ARA ET AL: "From Dietary Fiber to Host Physiology: Short-Chain Fatty Acids as Key Bacterial Metabolites", CELL, ELSEVIER, AMSTERDAM NL, vol. 165, no. 6, 2 June 2016 (2016-06-02), pages 1332 - 1345, XP029567379, ISSN: 0092-8674, DOI: 10.1016/J.CELL.2016.05.041
- PEDRO GONÇALVES ET AL: "A Cross-Talk Between Microbiota-Derived Short-Chain Fatty Acids and the Host Mucosal Immune System Regulates Intestinal Homeostasis and Inflammatory Bowel Disease", HHS PUBLIC ACCESS AUTHOR MANUSCRIPT, vol. 24, no. 3, 16 February 2018 (2018-02-16), US, pages 558 - 572, XP055752811, ISSN: 1078-0998, DOI: 10.1093/ibd/izx029
- CAVAGLIERI C ET AL: "Differential effects of short-chain fatty acids on proliferation and production of pro- and anti-inflammatory cytokines by cultured lymphocytes", LIFE SCIENCE, PERGAMON PRESS, OXFORD, GB, vol. 73, 1 January 2003 (2003-01-01), pages 1683 - 1690, XP007919115, ISSN: 0024-3205
- MEIJER KEES ET AL: "Butyrate and other short-chain fatty acids as modulators of immunity: what relevance for health?", CURRENT OPINION IN CLINICAL NUTRITION AND METABOLIC CARE NOV 2010, LIPPINCOTT WILLIAMS & WILKINS, US, vol. 13, no. 6, 1 November 2010 (2010-11-01), pages 715 - 721, XP009167656, ISSN: 1473-6519, DOI: 10.1097/MCO.0B013E32833EEBE5
- STÉ PHANE ET AL: "Butyrate Strongly Inhibits In Vitro Stimulated Release of Cytokines in Blood", DIGESTIVE DISEASES AND SCIENCE, 1 April 2002 (2002-04-01), pages 921 - 928, XP055754414, Retrieved from the Internet <URL:https://link.springer.com/article/10.1023%2FA%3A1014781109498#rightslink> [retrieved on 20201126]

## Description

### Technical Field of the Invention

This invention relates to the short-chain fatty acid pentanoate in a method for the activation of immune cells as defined in the appended claims. Valerate is a synonym for pentanoate. The invention involves improving the cultivation of chimeric antigen receptor (CAR) T cells by incubating them with short-chain fatty acid (SCFA) pentanoate after isolation from peripheral white blood cells. Those incubated peripheral white blood cells are reinjected to the same patient. The effect is that these cells are activated and the production of effector molecules in the patient is increased. This increases the chances of success of tumor therapy. This is illustrated by T-cells from mice that are transferred to mice with subcutaneous pancreatic tumors after the procedure. This type of cell treatment can be transferred to human T-cells and the improved treatment of pancreatic, lung, bladder, ovary, colon, skin, liver, brain and hematologic cancer as well as of infectious and autoimmune diseases.

This invention as defined in the appended claims also uses the short-chain fatty acid butyrate. It also uses a composition of short-chain fatty acid comprising pentanoate and/or butyrate.

This disclosure also relates to an improvement in the treatment of cancer, infectious diseases and immune cell-mediated diseases by means of cellular immune therapy (adoptive immune therapy). In particular, it relates to the use of pharmaceutically acceptable preparations of short-chain fatty acids. This disclosure also relates to the short-chain fatty acid butyrate and its use as enhancer for cellular immune therapy and anti-tumor therapy.

Cellular immune therapy (adoptive immune therapy) is an adoptive cell transfer (ACT) into a patient. The cells may have originated from the patient or from another individual. The cells are most commonly derived from the immune system with the goal of improving immune functionality and characteristics. In autologous cellular immune therapy, T cells are extracted from the said patient, genetically modified and cultured *in vitro* and returned to the same patient. Comparatively, allogeneic cellular immune therapies involve cells isolated and expanded from a donor separate from the patient receiving the cells. Cellular immune therapy is used for the treatment of patients suffering from cancer, infectious and immune cell-mediated diseases.

### State of the Art

Some commensal bacteria such as *Akkermansia muciniphila* and *Bifidobacterium* species are capable of enhancing anti-tumor immunity. However, the underlying molecular mechanisms and the contribution of diverse bacterial metabolites to these described anti-tumor effects remain obscure. We show here that the short-chain fatty acid (SCFA) pentanoate enhances the function of CD8⁺ cytotoxic T lymphocytes (CTLs). Pentanoate acts as a selective class I histone deacetylase (HDAC) inhibitor triggering an increase in histone H4 acetylation at the promoter regions of *Tbx21, Ifn*γ and *Eomes,* resulting in the enhanced production of effector molecules such as granzyme B and TNF-α in human and murine CTLs. Simultaneously, pentanoate promotes the long-term persistence and expansion of tumor-infiltrating CTLs. A broad screening approach revealed that among the commensal strains tested, only a few bacterial species exhibit a strong HDAC inhibitory capacity. Three commensals in particular, *Megasphaera massiliensis,* one of few human gut bacteria known to synthetize high levels of pentanoate, as well as two strong butyrate producers, *Faecalibacterium prausnitzii* and *Anaerostipes hadrus,* have a potent class I HDAC inhibitory activity. Notably, M. *massiliensis* significantly enhances the effector function of CD8⁺ T cells and promotes anti-tumor immunity.

The intestinal microbiota has been shown to directly impact on the efficacy of specific cancer immune therapies (Matson, V., Fessler, J., Bao, R., Chongsuwat, T., Zha, Y., Alegre, M.L., Luke, J.J., and Gajewski, T.F. (2018). The commensal microbiome is associated with anti-PD-1 efficacy in metastatic melanoma patients. Science 359, 104-108). Particularly, immune checkpoint inhibitory (ICI) therapy and adoptive cell therapy using tumor-specific CD8⁺ cytotoxic T lymphocytes (CTLs) can be influenced by the composition of intestinal microbiota (Wang, Y., Ma, R., Liu, F., Lee, S.A., and Zhang, L. (2018). Modulation of Gut Microbiota: A Novel Paradigm of Enhancing the Efficacy of Programmed Death-1 and Programmed Death Ligand-1 Blockade Therapy. Frontiers in immunology 9, 374. and Zitvogel, L., Ma, Y., Raoult, D., Kroemer, G., and Gajewski, T.F. (2018). The microbiome in cancer immunotherapy: Diagnostic tools and therapeutic strategies. Science 359, 1366-1370.). Recently, several studies have demonstrated that members of the gut microbiota are able to enhance the anti-tumor efficacy of PD-1 and CTLA4 blockade therapy (Tanoue, T., Morita, S., Plichta, D.R., Skelly, A.N., Suda, W., Sugiura, Y., Narushima, S., Vlamakis, H., Motoo, I., Sugita, K., et al. (2019). A defined commensal consortium elicits CD8 T cells and anti-cancer immunity. Nature 565, 600-605. and Vetizou, M., Pitt, J.M., Daillere, R., Lepage, P., Waldschmitt, N., Flament, C., Rusakiewicz, S., Routy, B., Roberti, M.P., Duong, C.P., et al. (2015). Anticancer immunotherapy by CTLA-4 blockade relies on the gut microbiota. Science 350, 1079-1084.).

*Akkermansia muciniphila* and some *Bifidobacterium* strains have been shown to modulate anti-tumor immune responses and improve ICI (Routy, B., Le Chatelier, E., Derosa, L., Duong, C.P.M., Alou, M.T., Daillere, R., Fluckiger, A., Messaoudene, M., Rauber, C., Roberti, M.P., et al. (2018). Gut microbiome influences efficacy of PD-1-based immunotherapy against epithelial tumors. Science 359, 91-97. and Sivan, A., Corrales, L., Hubert, N., Williams, J.B., Aquino-Michaels, K., Earley, Z.M., Benyamin, F.W., Lei, Y.M., Jabri, B., Alegre, M.L., et al. (2015). Commensal Bifidobacterium promotes antitumor immunity and facilitates anti-PD-L1 efficacy. Science 350, 1084-1089.). Furthermore, a defined commensal consortium (i.e. group of bacteria) consisting of 11 human bacterial strains elicited strong CD8⁺ T cell-mediated anti-tumor immunity in an experimental subcutaneous tumor model. This study has demonstrated that a mixture of human low-abundant commensals was able to substantially enhance the efficacy of ICI therapy in mice (Tanoue, T., Morita, S., Plichta, D.R., Skelly, A.N., Suda, W., Sugiura, Y., Narushima, S., Vlamakis, H., Motoo, I., Sugita, K., et al. (2019). A defined commensal consortium elicits CD8 T cells and anti-cancer immunity. Nature 565, 600-605. and Skelly, A.N., Sato, Y., Kearney, S., and Honda, K. (2019). Mining the microbiota for microbial and metabolite-based immunotherapies. Nature reviews. Immunology 19, 305-323.). So far, the exact underlying mechanisms causing the beneficial effects of commensal microbiota or microbiota-derived metabolites on anti-tumor immunity are not provided.

Relatively little is known about the role of soluble microbial metabolites in promoting anti-tumor immune responses. The major gut bacteria-derived metabolites, short-chain fatty acids (SCFAs) acetate, propionate and butyrate, have been shown to promote the expansion of Tregs, but also to improve the function of effector T cells (Arpaia, N., Campbell, C., Fan, X., Dikiy, S., van der Veeken, J., deRoos, P., Liu, H., Cross, J.R., Pfeffer, K., Coffer, P.J., and Rudensky, A.Y. (2013). Metabolites produced by commensal bacteria promote peripheral regulatory T-cell generation. Nature 504, 451-455. and Furusawa, Y., Obata, Y., Fukuda, S., Endo, T.A., Nakato, G., Takahashi, D., Nakanishi, Y., Uetake, C., Kato, K., Kato, T., et al. (2013). Commensal microbe-derived butyrate induces the differentiation of colonic regulatory T cells. Nature 504, 446-450. and Kespohl, M., Vachharajani, N., Luu, M., Harb, H., Pautz, S., Wolff, S., Sillner, N., Walker, A., Schmitt-Kopplin, P., Boettger, T., et al. (2017). The Microbial Metabolite Butyrate Induces Expression of Th1-Associated Factors in CD4+ T Cells. Frontiers in immunology 8, 1036. and Park, J., Goergen, C.J., HogenEsch, H., and Kim, C.H. (2016). Chronically Elevated Levels of Short-Chain Fatty Acids Induce T Cell-Mediated Ureteritis and Hydronephrosis. Journal of immunology 196, 2388-2400. and Park, J., Kim, M., Kang, S.G., Jannasch, A.H., Cooper, B., Patterson, J., and Kim, C.H. (2015). Short-chain fatty acids induce both effector and regulatory T cells by suppression of histone deacetylases and regulation of the mTOR-S6K pathway. Mucosal immunology 8, 80-93. and Smith, P.M., Howitt, M.R., Panikov, N., Michaud, M., Gallini, C.A., Bohlooly, Y.M., Glickman, J.N., and Garrett, W.S. (2013). The microbial metabolites, short-chain fatty acids, regulate colonic Treg cell homeostasis. Science 341, 569-573). Recently, two reports have highlighted the role of the SCFA butyrate in promoting the memory potential and antiviral *cytotoxic* effector functions of CD8⁺ T cells (Bachem, A., Makhlouf, C., Binger, K.J., de Souza, D.P., Tull, D., Hochheiser, K., Whitney, P.G., Fernandez-Ruiz, D., Dahling, S., Kastenmuller, W., et al. (2019). Microbiota-Derived Short-Chain Fatty Acids Promote the Memory Potential of Antigen-Activated CD8(+) T Cells. Immunity. and Trompette, A., Gollwitzer, E.S., Pattaroni, C., Lopez-Mejia, I.C., Riva, E., Pernot, J., Ubags, N., Fajas, L., Nicod, L.P., and Marsland, B.J. (2018). Dietary Fiber Confers Protection against Flu by Shaping Ly6c(-) Patrolling Monocyte Hematopoiesis and CD8(+) T Cell Metabolism. Immunity 48, 992-1005 e1008.). Our previous study revealed that the SCFA pentanoate (valerate) is also a bacterial metabolite synthetized in the gut of conventional but not of germ-free (GF) mice (Luu, M., Pautz, S., Kohl, V., Singh, R., Romero, R., Lucas, S., Hofmann, J., Raifer, H., Vachharajani, N., Carrascosa, L.C., et al. (2019). The short-chain fatty acid pentanoate suppresses autoimmunity by modulating the metabolic-epigenetic crosstalk in lymphocytes. Nature communications 10, 760.). Interestingly, dominant commensal bacteria are not able to produce pentanoate. Fecal concentrations of this SCFA are thus much lower than those of acetate, propionate and butyrate but relatively similar to physiological levels of the branched-chain fatty acids (BCFAs) isovalerate (isopentanoate) and isobutyrate (Koh, A., De Vadder, F., Kovatcheva-Datchary, P., and Backhed, F. (2016). From Dietary Fiber to Host Physiology: Short-Chain Fatty Acids as Key Bacterial Metabolites. Cell 165, 1332-1345.). Pentanoate is generated by thus far unidentified metabolic pathways, which may originate from the fermentation of either complex carbohydrates or dietary proteins by low-abundant commensals. In this invention, we investigated the influence of human commensals and their metabolites on CD8⁺ T cells and anti-cancer immunity. We found that particularly pentanoate and *Megasphaera massiliensis,* a recently classified pentanoate-producing bacterial species (Padmanabhan, R., Lagier, J.C., Dangui, N.P., Michelle, C., Couderc, C., Raoult, D., and Fournier, P.E. (2013). Non-contiguous finished genome sequence and description of Megasphaera massiliensis sp. nov. Standards in genomic sciences 8, 525-538. and Yuille, S., Reichardt, N., Panda, S., Dunbar, H., and Mulder, I.E. (2018). Human gut bacteria as potent class I histone deacetylase inhibitors in vitro through production of butyric acid and valeric acid. PloS one 13, e0201073.), induced the production of effector molecules as well as the longevity of CTLs, which enabled them to eliminate established tumors in mice.

The method to activate immune cells by gene transfer for use in cellular immune therapy is well known by a skilled person. The gene transfer can be performed in *vivo* or *in vitro.* Details are described in the publications Querques, I., Mades, A., Zuliani, C., Miskey, C., Alb, M., Grueso, E., Machwirth, M., Rausch, T., Einsele, H., et al. (2019). A highly soluble sleeping beauty transposase improves control of gene insertion (Nature Biotechnology 37, 1502-1512(2019), Agarwal, S., Hanauer, J. D.S., Frank, A. M., Reichert, V., Thalheimer, F. B., and Buchholz, C. J. (2020). In vivo Generation of CAR T cells Selectively in Human CD4+ Lymphocytes. Molecular Therapy 8, p1741-1932 and Agarwal, S., Weidner, T., Thalheimer F. B., and Buchholz C. J. (2019). In vivo generated human CAR T cells eradicate tumor cells. Oncoimmunology 8, e1671761 and Smith, T. T., Stephan, S. B, Moffett, H. F., McKnight, L. E., Ji, W., Reiman, D., Bonagofski, E., Wohlfahrt, M. E., Pillai, S. P. S., and Stephan, M. T. (2017). In situ programming of leukaemia-specific T cells using synthetic DNA nanocarries. Nature Nanotechnology 12, 813-820(2017) and Hudecek M, Schmitt TM, Baskar S, Lupo-Stanghellini MT, Nishida T, Yamamoto TN, Bleakley M, Turtle CJ, Chang WC, Greisman HA, Wood B, Maloney DG, et al. (2010) The B-cell tumor-associated antigen ROR1 can be targeted with T cells modified to express a ROR1-specific chimeric antigen receptor. Blood 25,116(22):4532-4541 and Monjezi R, Miskey C, Gogishvili T, Schleef M, Schmeer M, Einsele H, Ivics Z, Hudecek M. (2016) Enhanced CAR T-cell engineering using non-viral Sleeping Beauty transposition from minicircle vectors. Leukemia 31(1):186,194.

The administration route of *in vitro* activated immune cells for use in cellular immune therapy is well known by a skilled person. Immune cells for use in cellular immune therapy, which are *in vivo* activated, must not be administered to the patient because they are already in the said patient. Details are described in the publications Agarwal, S., Hanauer, J. D.S., Frank, A. M., Reichert, V., Thalheimer, F. B., and Buchholz, C. J. (2020). In Vivo Generation of CAR T cells Selectively in Human CD4+ Lymphocytes. Molecular Therapy 8, p1741-1932 and Agarwal, S., Weidner, T., Thalheimer F. B., and Buchholz C. J. (2019). In vivo generated human CAR T cells eradicate tumor cells. Oncoimmunology 8, e1671761 and Smith, T. T., Stephan, S. B, Moffett, H. F., McKnight, L. E., Ji, W., Reiman, D., Bonagofski, E., Wohlfahrt, M. E., Pillai, S. P. S., and Stephan, M. T. (2017). In situ programming of leukaemia-specific T cells using synthetic DNA nanocarries. Nature Nanotechnology 12, 813-820(2017).

### Task of the Invention

The task of the invention is to improve the production of effector molecules by peripheral white blood cell in a patient suffering from cancer (hematologic and oncologic tumors) or infectious diseases or immune-mediated diseases like autoimmune diseases and degenerative diseases. This will improve the outcome of cellular immune therapy in patients suffering from cancer (hematologic and oncologic tumors) or infectious diseases or immune cell-mediated diseases like autoimmune diseases and degenerative diseases.

### Solution of the Task

Claim 1 solves the task of the invention. The invention as defined in the appended claims describes a method for the cultivation of chimeric antigen receptor (CAR) T cells by incubating them with short-chain fatty acids (SCFAs) like pentanoate, butyrate or a composition of short-chain fatty acids comprising pentanoate and/or butyrate so that the chimeric antigen receptor (CAR) T cells are activated and the production of effector molecules is increased. The disclosure also involves improving the cultivation of T cells by incubating them with short-chain fatty acid (SCFA) pentanoate after isolation from peripheral white blood cells. Those incubated peripheral white blood cells are reinjected to the same patient. The effect is that these cells are activated and the production of effector molecules in the patient is increased. This increases the chances of success of tumor therapy.

Collectively, we disclose that low-abundant commensal bacterial species such as *M. massiliensis* and their selective metabolites such as pentanoate, rather than broadly distributed and abundant commensals, are used as specific microbial therapeutics for targeting human tumors.

We disclose a medical preparation for use in mammals, in particular in humans suffering from cancer (hematologic and oncologic tumors) or infectious diseases or immune-mediated diseases like autoimmune diseases and degenerative diseases, comprising at least one short-chain fatty acid (SCFA) like pentanoate, butyrate, characterised in that the at least one short-chain fatty acid (SCFA) activate immune cells and enhances the production of effector molecules.

The short-chain fatty acids like pentanoate and/or butyrate can be used as salts, for example as sodium salt (Na⁺-salt) or in another acceptable pharmaceutical preparation.

The T cells which are incubated with the short-chain fatty acid pentanoate are chimeric antigen receptor (CAR) T cells. The increased capacity of SCFA-treated CD8⁺ cytotoxic T lymphocytes (CTLs) and CAR T cells to eradicate tumor growth is mediated through integrated metabolic and epigenetic reprogramming of these cells. SCFAs acted on CTLs and CAR T cells by increasing the function of a central cellular metabolic sensor, mTOR, and via inhibition of class I histone deacetylase (HDAC) activity. This resulted in elevated production of effector molecules such as CD25, IFN-γ and TNF-α in CTLs and CAR T cells. Therefore, that specific microbial molecules are used for enhancing the efficacy of cancer immunotherapy with T cells.

In a preferred embodiment the chimeric antigen receptor (CAR) T cells which are incubated with the short-chain fatty acid pentanoate are co-incubated with the short-chain fatty acid butyrate. Pentanoate and butyrate induce the production of effector molecules and longevity of CTLs, which enables them to eliminate established tumors. Therefore, pentanoate and butyrate are effective biotherapeutics to enhance anti-tumor immunity.

In a preferred embodiment the bacterium *Megasphaera massiliensis* is used as a pentanoate and butyrate producing supplier. Therefore, the T cells which are incubated with the short-chain fatty acids pentanoate and/or butyrate can also be incubated with the bacterium *Megasphaera massiliensis,* because this bacterium produces pentanoate and butyrate.

The short-chain fatty acid pentanoate is used as a pharmaceutical active compound for the treatment of tumors.

In a reference aspect of the disclosure the short-chain fatty acids pentanoate and/or butyrate are used as a pharmaceutical active compound for the treatment of tumors.

In a preferred reference aspect of the disclosure the short-chain fatty acids pentanoate and/or butyrate are used as a pharmaceutical active compound for the treatment of a pancreatic cancer.

In order to improve the outcome of cellular immune therapy in patients suffering from cancer (hematologic and oncologic tumors) or infectious diseases or immune-mediated diseases like autoimmune diseases and degenerative diseases, immune cells are incubated with short-chain fatty acids, which activates said immune cells. The activation of the immune cells by short-chain fatty acids can be performed *in vivo* or *in vitro.*

*In vivo* means that the short-chain fatty acids are administered to the immune cells inside a patient in an appropriate way, for example but not limited to per os, intravenous, intraperitoneal, which activates said immune cells inside the patient.

*In vitro* means that the short-chain fatty acids are administered to the immune cells outside a patient, which activates said immune cells outside the patient. Said activated immune cells are administered to the patient in an appropriate way, for example but not limited to per os, intravenous, intraperitoneal.

The invention as defined in the appended claims comprises a method for the activation of chimeric antigen receptor (CAR) T cells by incubating them with at least one short-chain fatty acid so that the immune cells are activated so that they can increase the production of effector molecules. This method is also characterized in that the short-chain fatty acid comprises pentanoate or a pharmaceutical acceptable derivative thereof. This method according is also in that the short-chain fatty acid comprises pentanoate and butyrate or pharmaceutical acceptable compositions thereof. This method is also characterized in that the at least one short-chain fatty acid is produced by at least one species of bacteria. This method is also characterized in that the at least one short-chain fatty acid is produced by the bacterium *Megasphaera massiliensis.* This method is also characterized in that the at least one short-chain fatty acid is produced by a group of bacteria comprising at least the bacteria *Megasphaera massiliensis, Megasphaera elsdenii, Faecalibacterium prausnitzii* and *Anaerostipes hadrus.*

The disclosure comprises the use of the activated immune cells for the treatment of tumors, immune mediated diseases, degenerative diseases and infectious diseases characterized in that the at least one short-chain fatty acid enhances a cellular immune therapy. The disclosure comprises the use of the activated immune cells for the treatment of tumors, immune mediated diseases, degenerative diseases and infectious diseases characterized in that the tumor is a pancreatic tumor.

The disclosure also comprises a method for the cultivation of T cells by incubating them with short-chain fatty acid pentanoate so that the T cells are activated and the production of effector molecules is increased.

### Embodiments

The disclosure describes the improvement of cellular immune therapy by enhancing the activation of immune cells and thus by increasing the production of effector molecules in the patient. This leads to a better anti-cancer medication or medication which regulates the immune system. In a preferred embodiment the short-chain fatty acids pentanoate and/or butyrate activate CAR T cells. In another reference aspect of the present disclosure short-chain fatty acids pentanoate and/or butyrate activate NK cells, γδ T cells, B lymphocytes and NK-T cells. In a preferred reference aspect of the present disclosure the short-chain fatty acids pentanoate and/or butyrate activate CD8 T cells and cytotoxic T cells (CTLs).

In one reference aspect of the present disclosure the short-chain fatty acids pentanoate and/or butyrate activate human CD8 T cells with endogenous T cell receptors, in another reference aspect of the present disclosure the short-chain fatty acids pentanoate and/or butyrate activate human CD8 T cells with transgenic T cell receptors, and in reference aspect of the present disclosure the short-chain fatty acids pentanoate and/or butyrate activate human CD8 T cells with synthetic receptors. Synthetic receptors can be chimeric antigen receptors (CARs) with variable intracellular signaling domain. In one preferred embodiment a ROR1-specific CAR is used.

The invention as defined in the appended claims describes the use of short-chain fatty acids to treat immune cells. The invention as defined in the appended claims uses pentanoate as short-chain fatty acid. The invention as defined in the appended claims also uses butyrate as short-chain fatty acid. In a preferred embodiment the invention as defined in the appended claims uses pentanoate in combination with butyrate as short-chain fatty acids. Pentanoate can also be combined with other short-chain fatty acids (SCFAs) including but not limited to acetate, propionate, valproate.

Immune cells are treated with short-chain fatty acids. In a preferred embodiment the short-chain fatty acid is pentanoate or a salt thereof. In another preferred embodiment the short-chain fatty acid is pentanoate or a salt thereof in combination with one or more other short-chain fatty acids or salts thereof. In another preferred embodiment the short-chain fatty acid is pentanoate or a salt thereof in combination with butyrate or a salt thereof.

Short-chain fatty acids can be obtained by cultures of bacteria. In a preferred embodiment a culture of *Megasphaera massiliensis* produces short-chain fatty acids. These short-chain fatty acids are obtainable from the supernatant. Cultures of *Megasphaera massiliensis* produce a combination of short-chain fatty acids, including pentanoate and butyrate. Short-chain fatty acids can also be obtained by extraction of valerian (*Valeriana officinalis*)*.* Short-chain fatty acids can also be obtained by chemical synthesis.

In a reference aspect of the present disclosure the activation of immune cells for use in cellular immune therapy is performed as follows: A culture of *Megasphaera massiliensis* is administered to the patient, for example *per os.* In this reference aspect of the present disclosure the patient's own immune cells are activated in *vivo* inside the patient after the patient was administered the culture of *Megasphaera massiliensis,* which produces one or more short-chain fatty acids inside the patient.

The disclosure is useful to improve the anti-cancer treatment in all kinds of cancer, including hematologic (including but not limited to leukemia, lymphoma, myeloma) and oncologic cancers (including but not limited to cancers of the pancreas, skin, lung, bladder, colon, brain, testis, ovaries and breast). The disclosure is also useful to treat immune-mediated disease like autoimmune diseases (including but not limited to psoriasis, lupus erythematosus, myasthenia gravis, rheumatoid arthritis) or degenerative diseases (including but not limited to multiple sclerosis). The disclosure as defined in the appended claims is also useful to treat infectious diseases (including but not limited to viral infections).

Preferably the disclosure as defined in the appended claims is suitable for treating diseases involving immune cells targeting one or more antigens selected from the group including but not limited to: CD19, CD20, CD22, CD33, BCMA, CD123, SLAMF7, CD138, CD38, CD70, CD44v6, CD56, EGFR, ERBB2, Mesothelin, PSMA, FAP, 5T4, FLT-3, MAGEA, MEGAB, GAGE1, SSX, NY-ESO-1, MAGEC1, MAGEC2, CTp11/SPANX, XAGE1/ GAGED, SAGE1, PAGE5, NA88, IL13RA1, CSAGE, CAGE, HOM-TES-85, E2F-like/ HCA661, NY-SAR-35, FTHL17, NXF2, TAF7L, FATE1, ROR-1, ROR-2, Integrins, Siglecs, cancer-testes antigens, neoantigens.

The administration route of *in vitro* activated immune cells for use in cellular immune therapy is well known by a skilled person.

In a reference aspect of the present disclosure the patient's own immune cells are activated *in vivo* inside the patient after the patient was administered one or more short-chain fatty acids.

In a preferred reference aspect of the present disclosure the immune cells are transferred to the same patient after they were activated by *in vitro* incubation with one or more short-chain fatty acids.

In another reference aspect of the present disclosure immune cells of a healthy person are transferred to a patient after they were activated by *in vitro* incubation with one or more short-chain fatty acids.

In another reference aspect of the present disclosure immune cells of a healthy person are activated *in vivo* inside the said person after the said person was administered one or more short-chain fatty acids. After this activation the activated immune cell are collected from this person and transferred to the patient.

In one embodiment the gene transfer for receptor constructs is conducted ex *vivo.* In another embodiment the gene transfer for receptor constructs is conducted in *vivo.* In this case the gene transfer can be conducted by nano-particle transposon technology or by viral gene transfer technology. These techniques are well known to skilled persons. Details are described in the publications Agarwal, S., Hanauer, J. D.S., Frank, A. M., Reichert, V., Thalheimer, F. B., and Buchholz, C. J. (2020). In Vivo Generation of CAR T cells Selectively in Human CD4+ Lymphocytes. Molecular Therapy 8, p1741-1932 and Agarwal, S., Weidner, T., Thalheimer F. B., and Buchholz C. J. (2019). In vivo generated human CAR T cells eradicate tumor cells. Oncoimmunology 8, e1671761 and Smith, T. T., Stephan, S. B, Moffett, H. F., McKnight, L. E., Ji, W., Reiman, D., Bonagofski, E., Wohlfahrt, M. E., Pillai, S. P. S., and Stephan, M. T. (2017). In situ programming of leukaemia-specific T cells using synthetic DNA nanocarries. Nature Nanotechnology 12, 813-820(2017).

**In** one embodiment the short-chain fatty acid or acids is/are used during the manufacturing of the immune cells, i.e. before the immune cells are administered to the patient. **In** another reference aspect of the present disclosure the short-chain fatty acid or acids is/are used after the administration of the immune cells to the patient. **In** another embodiment the short-chain fatty acid or acids is/are used during the manufacturing of the immune cells, i.e. before the immune cells are administered to the patient and after the administration of the immune cells to the patient.

**In** one embodiment autologous immune cells are activated by the short-chain fatty acid or acids, i.e. immune cells of said patient are activated. **In** another embodiment allogenic immune cells are activated by the short-chain-fatty acid or acids, i.e. immune cells of another person are activated and administered to a patient.

In one embodiment the short-chain fatty acid or acids is/are added directly to cell culture medium. In this case the short-chain fatty acid or acids is/are added during the manufacturing of the immune cells or after the manufacturing of the immune cells. In another reference aspect of the present disclosure the short-chain fatty acid or acids is/are added systemically to the patients in an acceptable pharmaceutical composition. Acceptable pharmaceutical compositions comprise for example solutions, pills, salts, drinks which can be administered orally or systemically. In another embodiment the short-chain fatty acid or acids is/are added directly to cell culture medium and systemically to the patients in an acceptable pharmaceutical composition. In another reference aspect of the present disclosure the short-chain fatty acid or acids is/are added by administration of short-chain fatty acid-producing bacteria to the patient. In this case these bacteria produce the short-chain fatty acid or acids inside the patients and therefore the activation of the immune cells takes place inside the patient. In another embodiment the short-chain fatty acid or acids is/are added by using the supernatant of bacteria culture medium. Preferably these bacteria are *Megasphaera massiliensis.*

The activation of immune cells can be performed by adding short-chain fatty acid or acids or by adding bacterial culture supernatant containing short-chain fatty acid or acids.

In one reference aspect of the present disclosure the activation of immune cells can be performed by injection of bacterial culture supernatant containing short-chain fatty acid or acids in patients during treatment with cell products. In another reference aspect of the present disclosure the activation of immune cells can be performed by injection of short-chain fatty acid or acids in patients during treatment with cell products.

In one embodiment short-chain fatty acid or acids produced by *Megasphaera massiliensis* are used to activate immune cells. In another embodiment short-chain fatty acid or acids produced by *Megasphaera massiliensis* and other bacteria are used to activate immune cells. In this case a group (consortium) of bacteria is used to produce short-chain fatty acid or acids (e. g. co-administration with *Megasphaera elsdenii, Faecalibacterium prausnitzii* and *Anaerostipes hadrus*). This consortium can also be obtained from the microbiome of the patient or from the microbiome of a different person. This person can be a healthy person, a person with the same or a different disease. For example, the consortium can be obtained from stool samples. In one embodiment this stool samples are obtained from the patient. In another embodiment this stool samples are obtained from good responders of the same treatment of the same disease with the desired therapeutic outcome.

The activation of immune cells by adding short-chain fatty acid or acids can be performed once or sequentially, preceeding, concurrent to and/ or after CAR T cell therapy. It is also possible to perform the CAR T cell-therapy once and to administer short-chain fatty acid or acids to the patient recurrently. In another reference aspect of the present disclosure CAR T cell therapy is performed once or recurrently and bacterial supernatants containing short-chain fatty acids or a bacterial consortium producing short-chain fatty acids are administered once or recurrently.

In a preferred reference aspect of the present disclosure the immune cell treatment is used in the context of autologous and/ or allogenic hematopoetic stem cell transplantation.

In one reference aspect of the present disclosure short-chain fatty acid treatment is administered concurrently to CAR T cell therapy and short-chain fatty acids are administered repeatedly (daily, weekly, monthly, quarterly). In a more preferred reference aspect of the present disclosure the administration is performed weekly.

In another reference aspect of the present disclosure short-chain fatty acid treatment is performed to modulate the tumor cells and the tumor microenvironment. Administration of short-chain fatty acids is used to modulate tumor features including but not limited to growth, signalling, escape mechanisms and antigen presentation.

### Modulation of CD8⁺ T lymphocytes by gut microbiota-derived short-chain fatty acids.

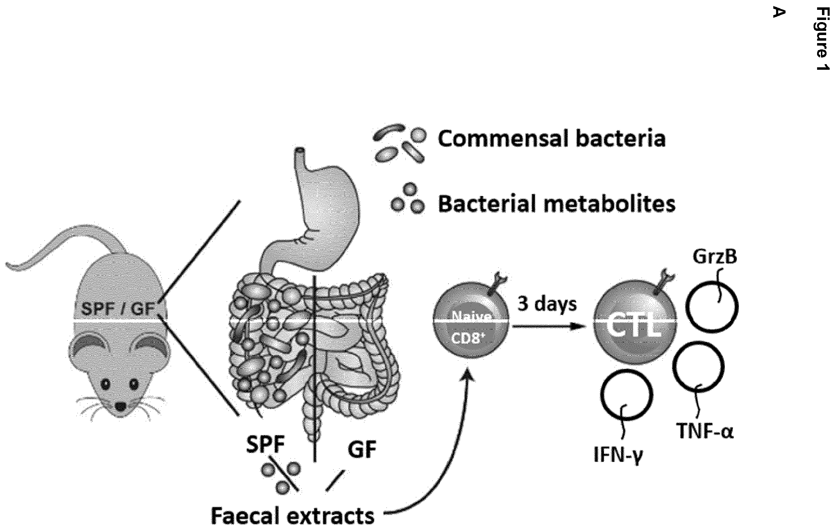

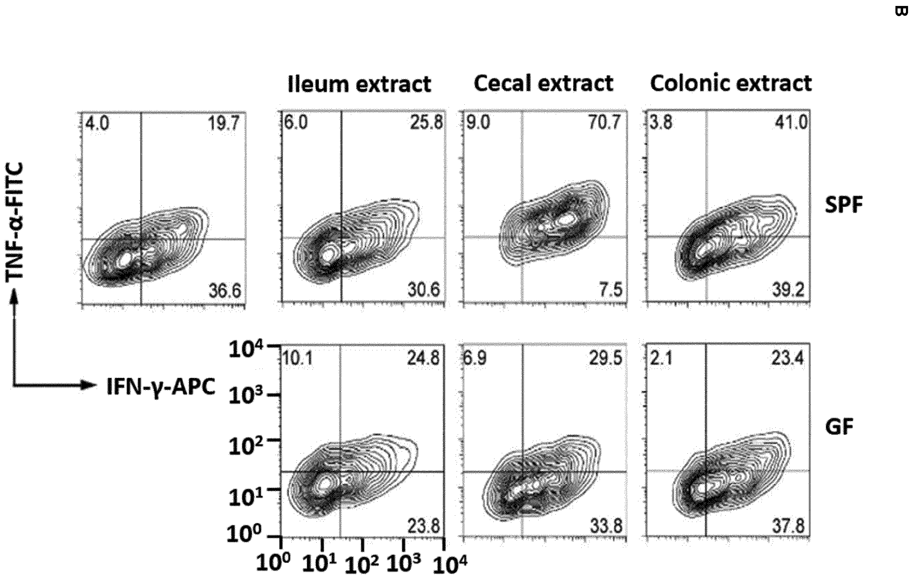

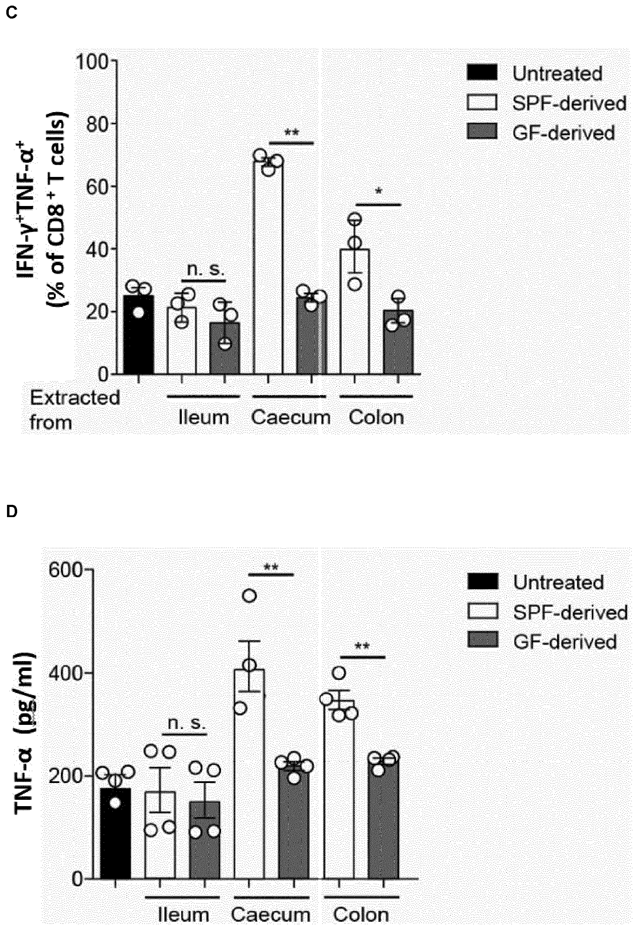

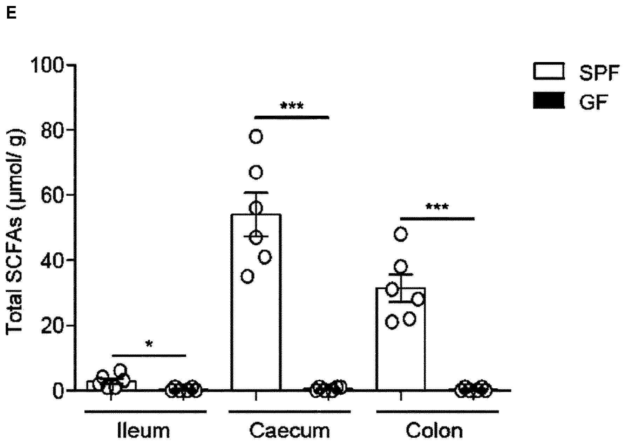

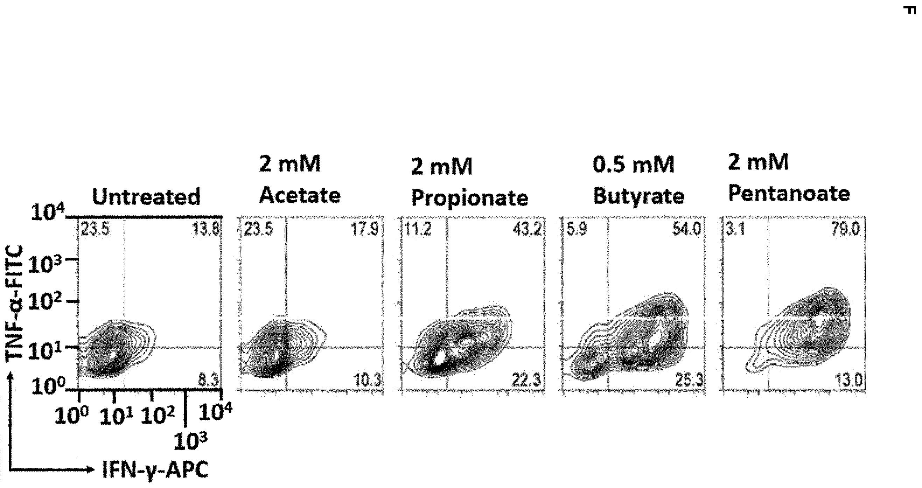

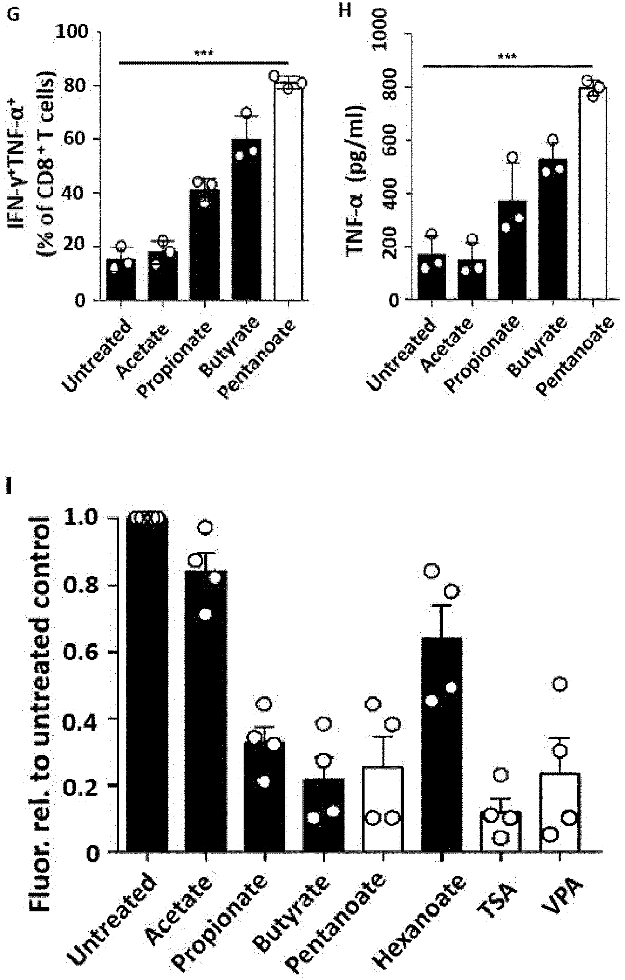

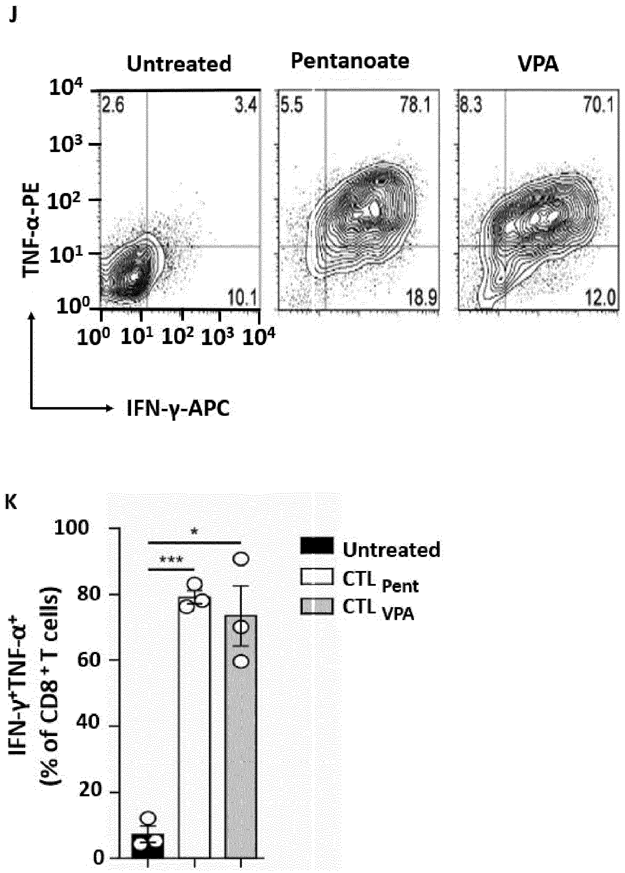

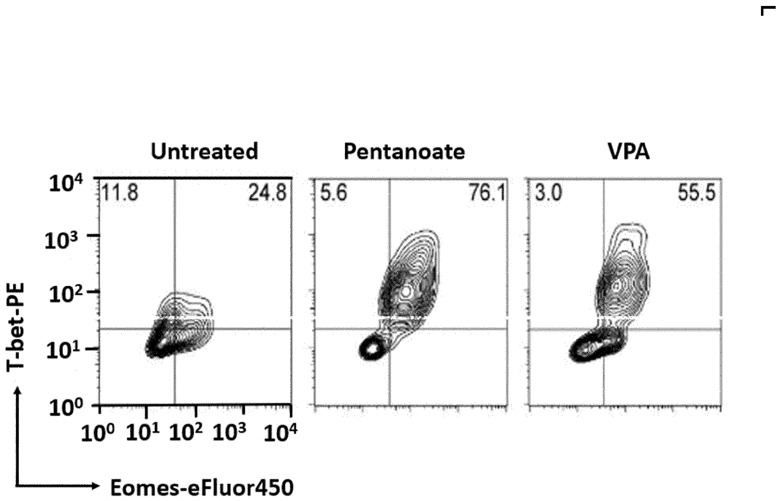

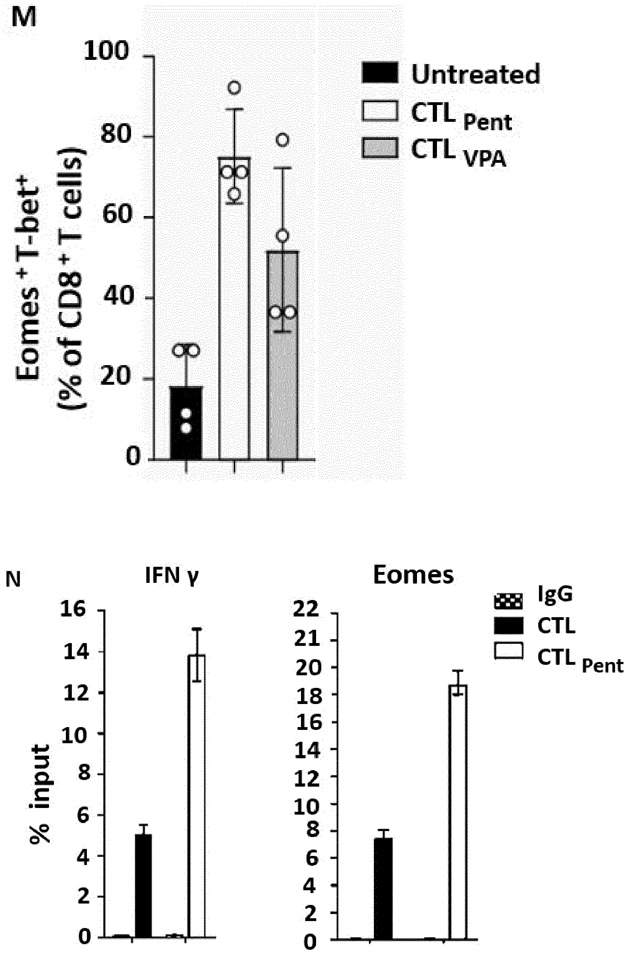

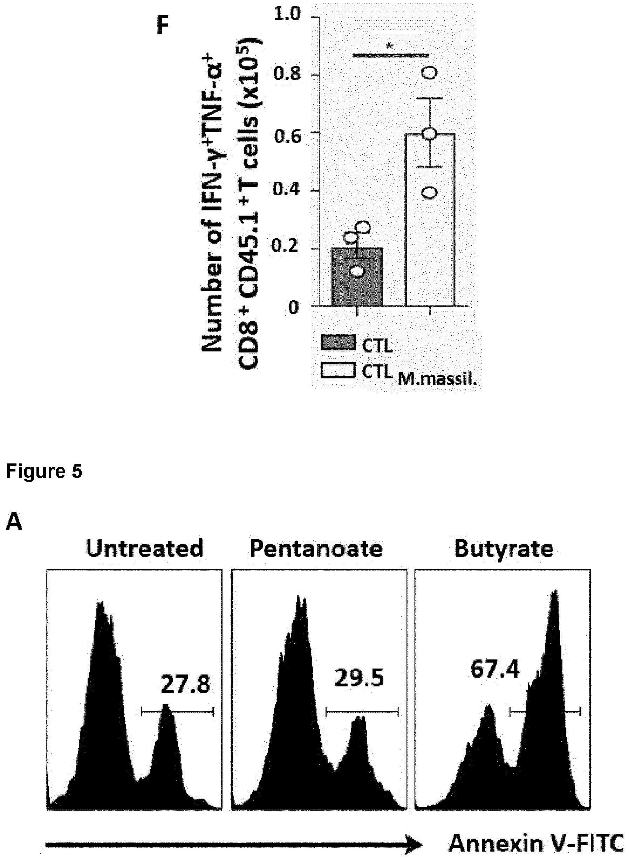

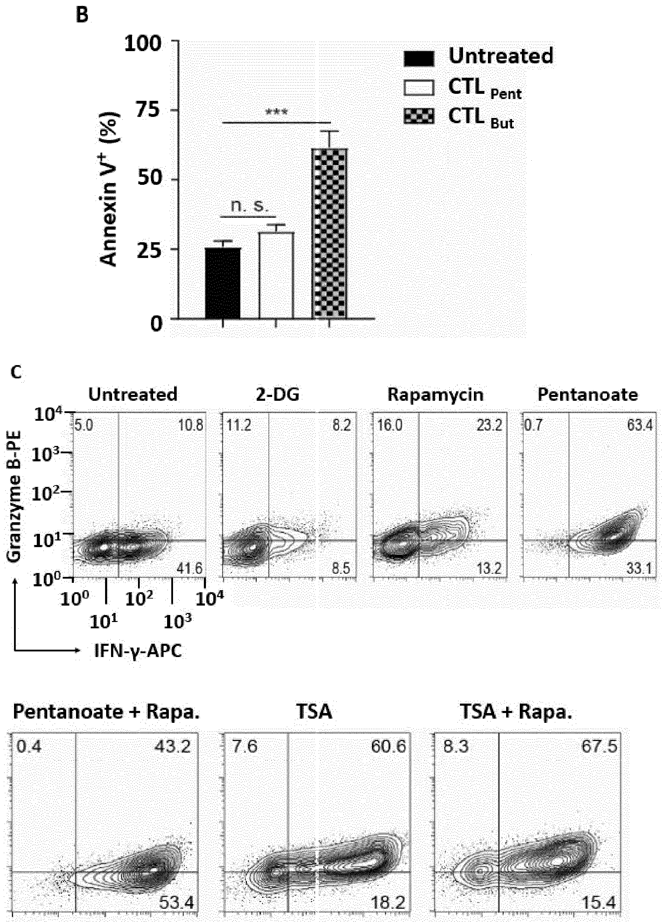

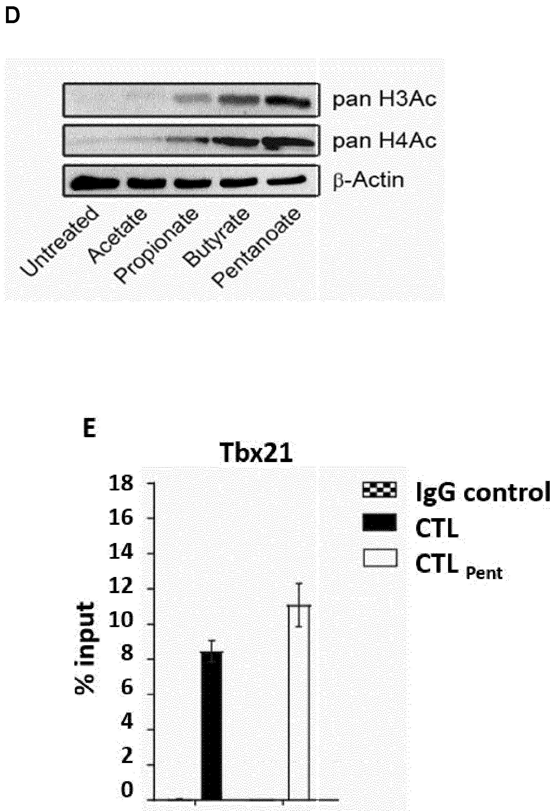

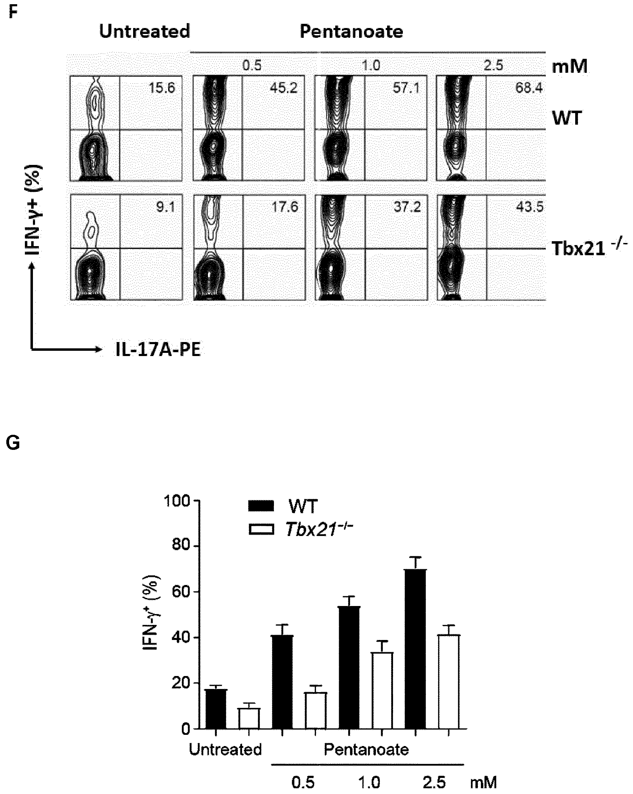

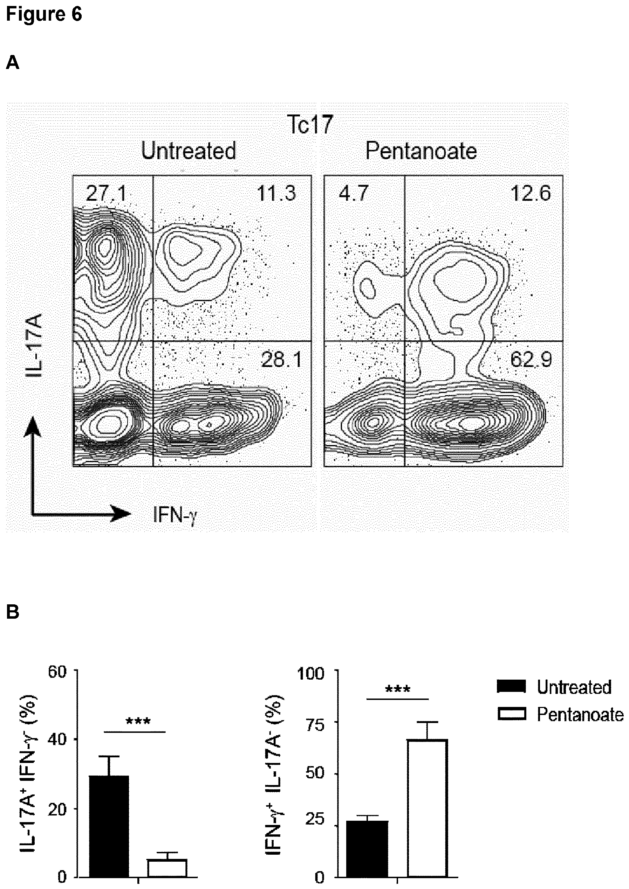

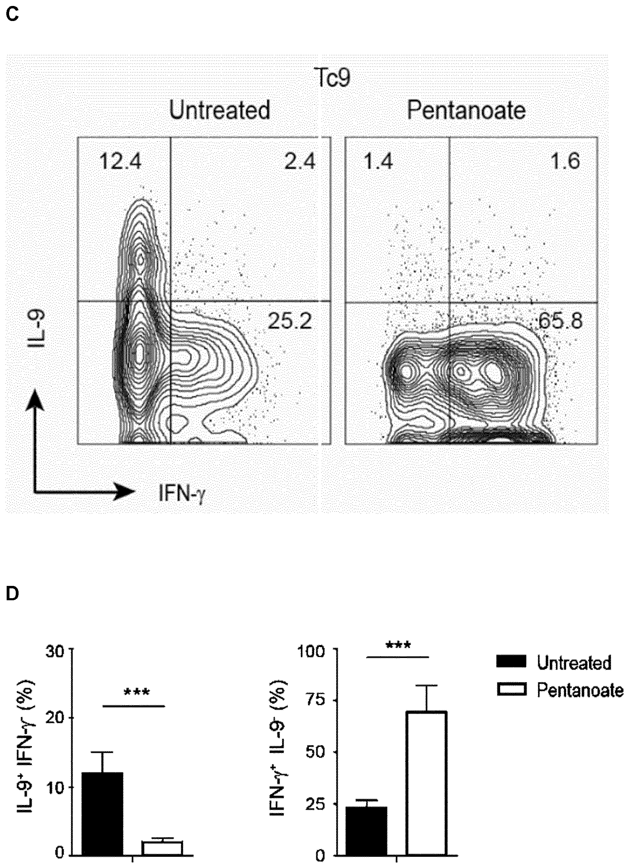

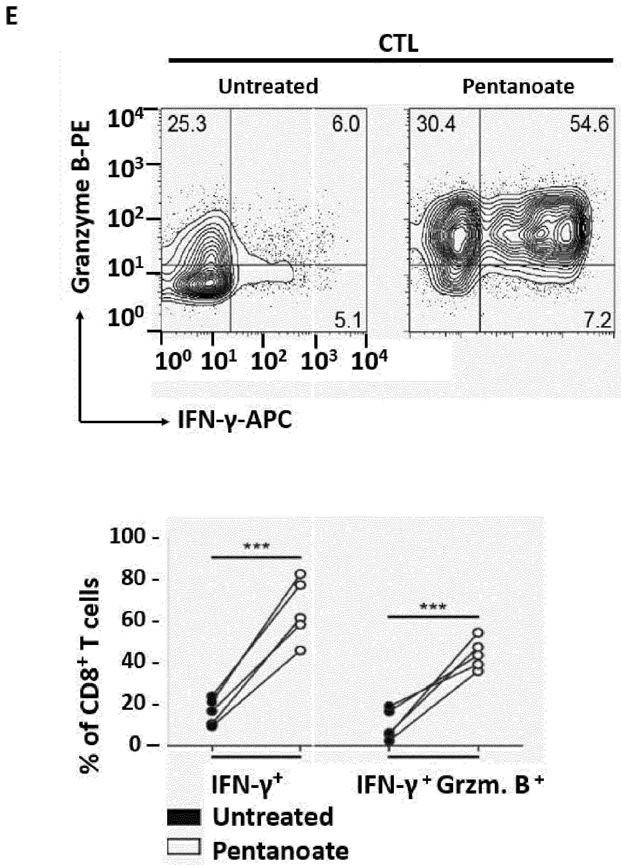

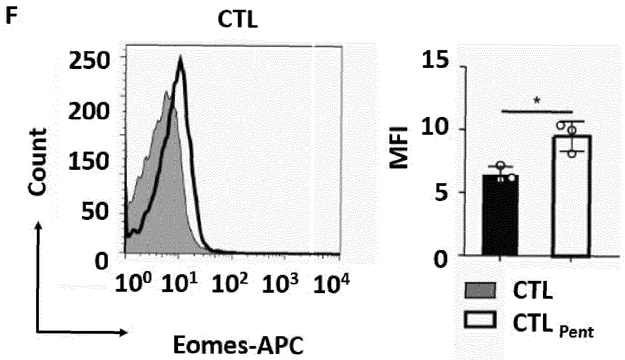

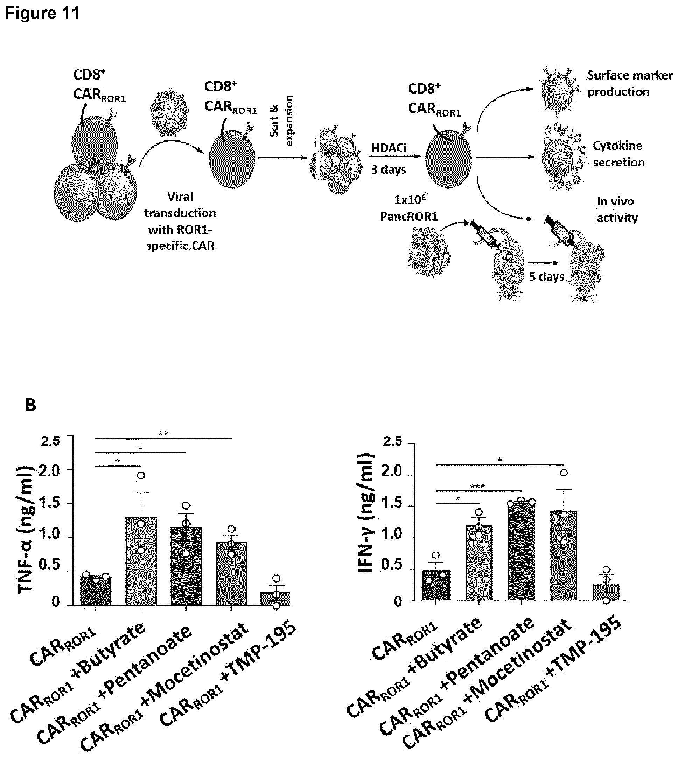

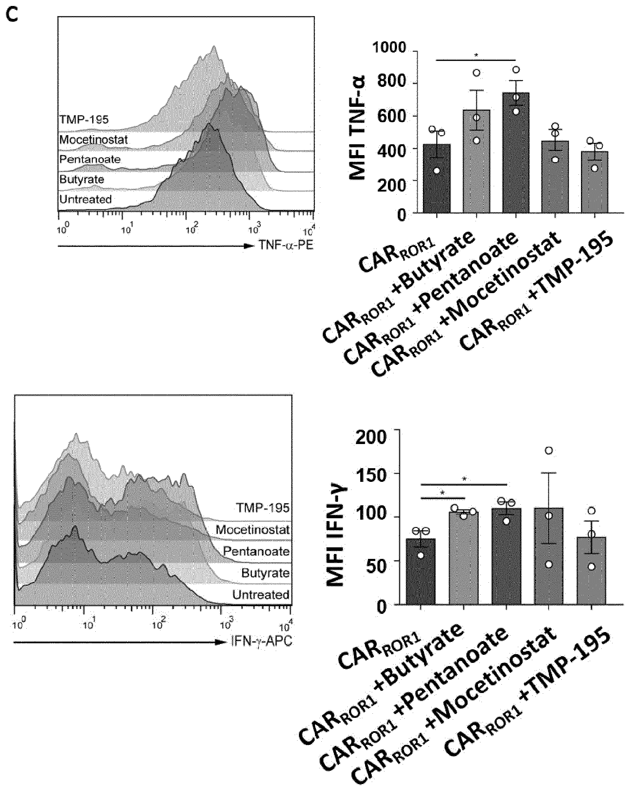

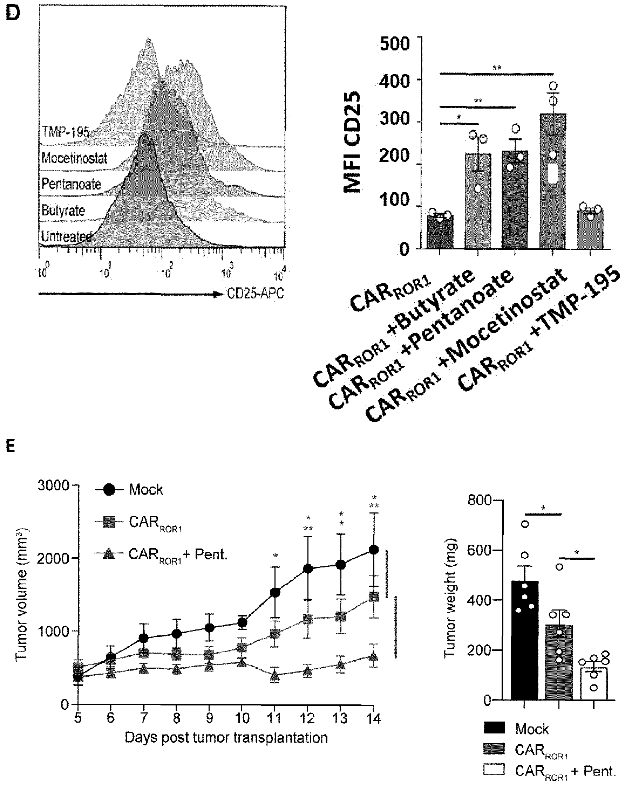

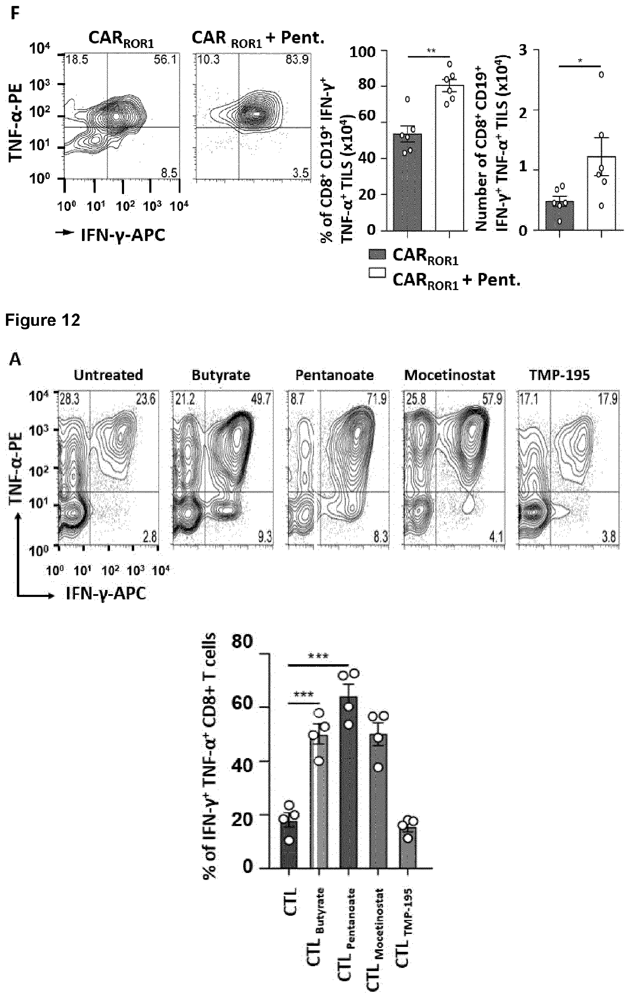

A recent study has shown that IFN-γ-secreting CD8⁺ T cells are present at high percentages in the intestine of specific pathogen-free (SPF) but not in that of GF mice. We hypothesized that not only bacteria themselves but also soluble, diffusible microbial mediators may directly impact on CD8⁺ T cells. Indeed, the water-soluble extracts from the luminal content of the colon, and particularly the caecum, of SPF mice exhibited strong effects on IFN-γ and TNF-α production in CTLs. In contrast, the soluble fraction of the intestinal luminal content derived from GF animals did not have any impact on the production of effector molecules by CTLs (Figure 1, A-D). Collectively, this analysis demonstrates that SPF-derived luminal content of the caecum and the colon contains bacterial metabolites that influence the expression of CTL-associated cytokines. Interestingly, the distribution and the fecal signature of microbial SCFAs corresponds to the observed phenotype. SCFAs such as acetate, propionate and butyrate are water-soluble and diffusible metabolites reaching their peak concentrations in the caecum and decrease from the proximal to the distal colon. They are not present in the intestinal lumen of GF mice, regardless of the compartment, and are hardly detectable in the small intestine of SPF mice (Figure 1E). In order to elucidate the role of SCFA-mediated effects on CD8⁺ T cells, we cultivated CTLs under suboptimal conditions in the presence of acetate, propionate, butyrate or pentanoate. We found that pentanoate and butyrate triggered a substantial increase in frequencies of TNF-α⁺ IFN-γ⁺ cells and secretion of TNF-α by CTLs (Figure 1, F-H). As already low concentrations of butyrate but not that of pentanoate induced apoptosis in CTLs (Figure 5, A and B), we decided to perform the further functional analysis with the latter SCFA. Together, these data suggest that commensal-derived molecules such as SCFAs are able to enhance the production of IFN-γ and TNF-α in CD8⁺ T cells. It is known that the glycolytic metabolic pathway promotes IFN-γ expression and T cell effector function. In line with these findings, the inhibition of glycolysis by the glucose analog 2-deoxyglucose (2-DG) or the mTOR complex (which promotes glycolytic metabolism in effector T cells) by rapamycin led to a reduction in IFN-γ production in CTLs (Figure 5 C). Although microbial SCFAs can be utilized by T cells for their metabolic demand to increase activity of the mTOR pathway and enhance glycolysis and oxidative phosphorylation, our data suggest that the histone deacetylase (HDAC) inhibitory activity of SCFAs rather than their metabolic input promotes the robust expression of effector molecules such as IFN-γ, TNF-α and granzyme B. Similar to pentanoate, the pan-HDAC inhibitor trichostatin A (TSA) substantially enhanced the percentages of granzyme B⁺IFN-γ⁺ CD8⁺ T cells as compared to control CTLs. Notably, this strong effect of TSA and pentanoate could not be reversed by treating the cells with rapamycin (Figure 5C). Western blot analysis showed an increased acetylation of histones H3 and H4 after treatment of T lymphocytes with propionate, butyrate and pentanoate (Figure 5D). Furthermore, CTL-derived cell lysates exposed to propionate, butyrate and pentanoate, but not to acetate and hexanoate, displayed a strong reduction of HDAC activity (Figure 11). Valproate (2-propylpentanoate) is a synthetic branched SCFA derived from pentanoate with a strong HDAC inhibitory activity (Figure 11). We observed that, similar to pentanoate, valproate potently enhanced the expression of both TNF-α and IFN-γ in CTLs (Figure 1J and 1K). Furthermore, both pentanoate and valproate strongly induced the CTL-related transcription factors *T-bet* and *Eomes* in CD8⁺ T cells (Figure 1, L and M). We next investigated if acetylation of H4 was increased at CTL-characteristic loci (*Ifn*γ, *Tbx21* and *Eomes*) after treatment of CTLs with pentanoate. Indeed, pentanoate was able to enhance H4 acetylation at the promoter region of *Ifn*γ, *Tbx21* and *Eomes* (Figure 1N and Figure 5E). Moreover, T-bet-deficient CTLs showed a partially defective production of IFN-γ after treatment with pentanoate (Figure 5, F and G). These results suggest that the HDAC inhibitory activity of pentanoate modulates the production of several effector molecules in CTLs. Due to the high level of heterogeneity and plasticity within T lymphocytes, we next investigated possible effects of pentanoate on other CD8⁺ T cell subtypes. We observed that pentanoate treatment of recently described subsets, Tc9 and Tc17 cells, induced a phenotypical switch towards IFN-γ-producing CTLs (Figure 6, A-D). To gain further insight into possible therapeutic strategies, we next investigated the impact of pentanoate on human CD8⁺ T lymphocytes.

Our data obtained with human T cells suggest that pentanoate could be of a therapeutically potential, especially for CAR therapy, as this microbial SCFA was able to induce CTL phenotype in human CD8⁺ T lymphocytes by enhancing the expression of granzyme B, IFN-γ and *Eomes* (Figure 6, E and F).

### Adoptive transfer of pentanoate-treated CTLs enhances anti-cancer immunity.

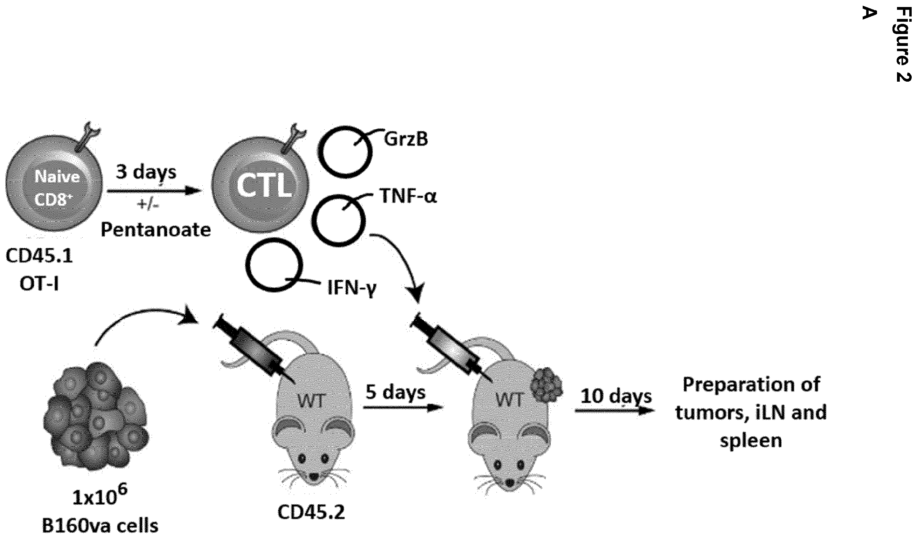

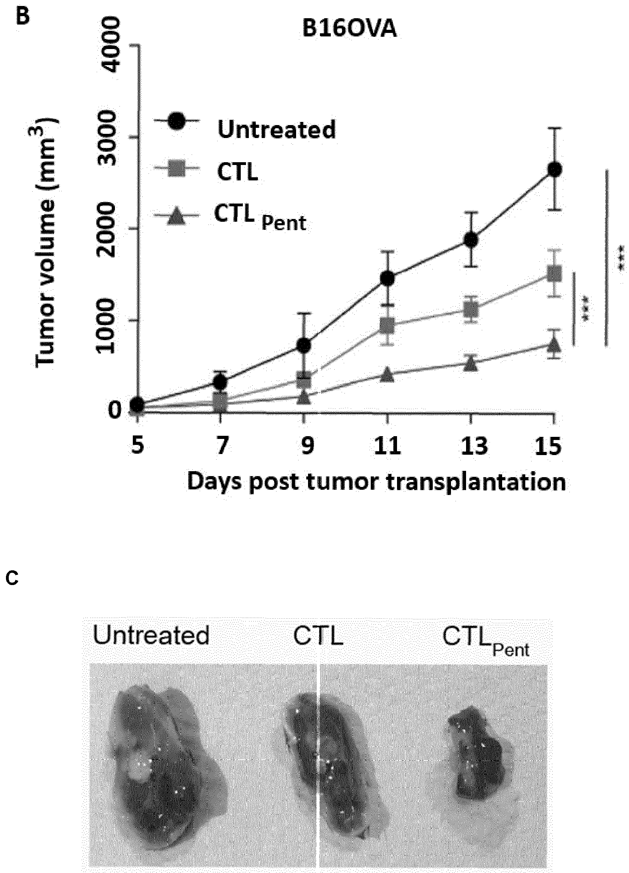

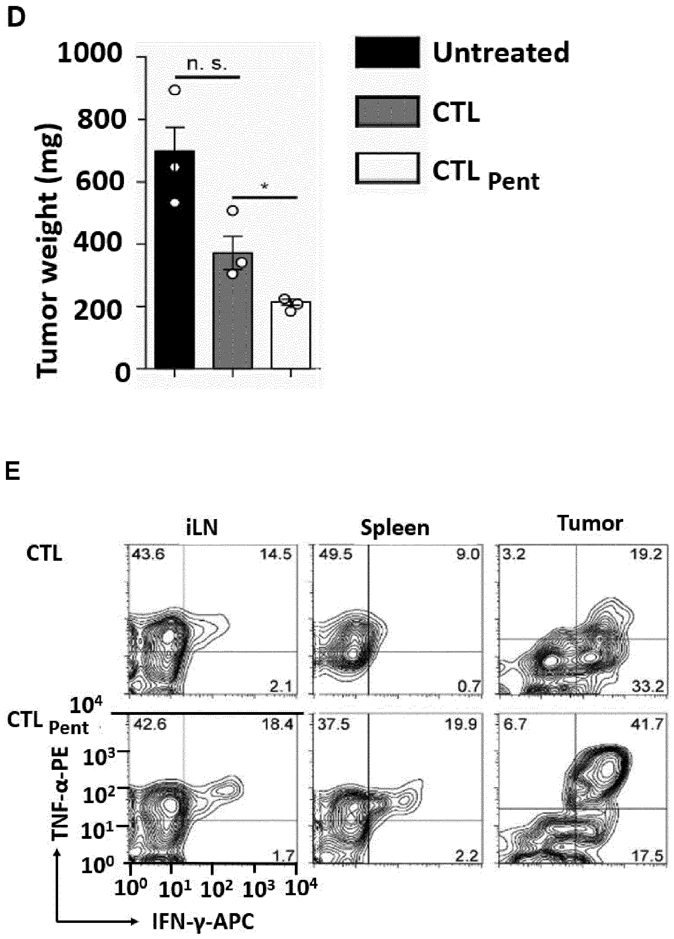

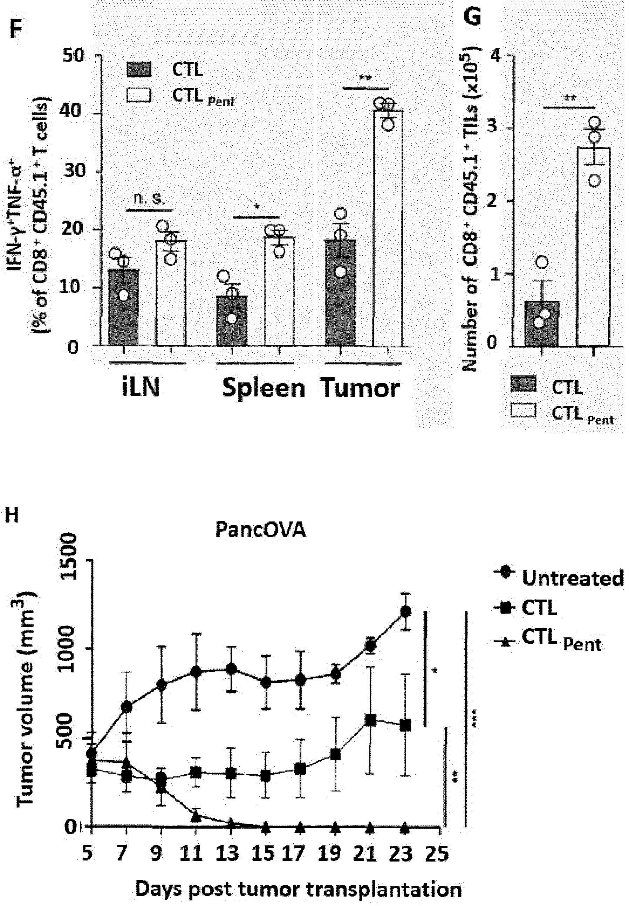

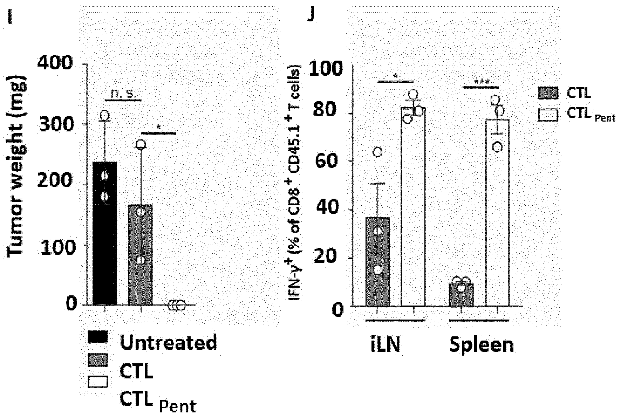

We next sought to determine if pentanoate-treated CTLs are superior to control counterparts in combating established tumors. We tested this hypothesis in two different subcutaneous (s.c.) tumor models. We injected s.c. B16OVA melanoma cells into CD45.2⁺ mice and transferred either control or pentanoate-treated CD45.1⁺ OT-I CTLs into recipient animals on day 5 after tumor injection. The anti-tumor immunity mediated by antigen-specific CTLs was significantly improved after pre-treatment with pentanoate, as shown by decreased tumor volume and weight (Figure 2, A-D). On day 10 after adoptive transfer of CTLs, we detected a higher number of pentanoate-treated cells expressing more effector cytokines TNF-α and IFN-γ in comparison to control OT-I CTLs in tumors, draining LNs and spleen (Figure 2, E-G). Furthermore, the anti-tumor effects mediated by treatment of CTLs with pentanoate were tested in aggressive pancreatic tumors expressing OVA protein (*OVA-expressing Panc02 cells,* PancOVA). To this end, 1.5 x 10⁶ PancOVA cells were injected s.c. into CD45.2⁺ mice. Notably, the transfer of low numbers (7.5 x 10⁵) of pentanoate-treated CD45.1⁺ OT-I CD8⁺ T lymphocytes, but not of control CTLs, was sufficient to completely eliminate the growth of PancOVA cells in recipient animals (Figure 2, H-I). While the control CTLs were hardly found on day 23 after tumor inoculation, we observed a persistence of pentanoate-treated CTLs with high IFN-γ production within draining LNs and spleen of recipient mice even 10 days after eliminating tumor cells (Figure 2J). These data suggest that pentanoate may be therapeutically used for adoptive cell therapy to target human tumors. One area of application is CAR therapy.

### Pentanoate-producing bacterium Megasphaera massiliensis enhances anti-tumor activity of CD8⁺ CTLs.

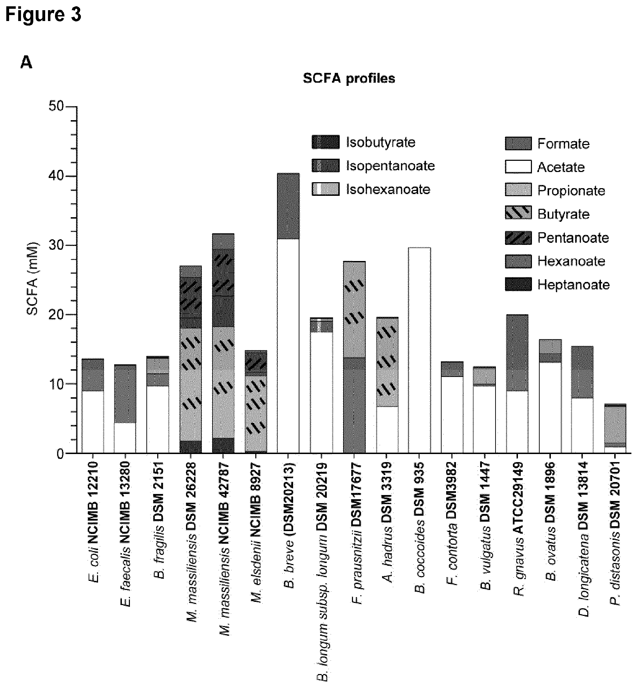

We recently showed that a human gut-isolated bacterial strain *Megasphaera massiliensis* is able to produce high levels of pentanoate. By broadly screening a panel of human commensals for their SCFA production profiles, we were not able to detect any significant pentanoate production in any of the strains tested, suggesting that mainly low-abundant strains in the gut may be capable of generating this specific SCFA. When compared to 14 bacterial abundant species, which represent proportional distribution of the most common phyla in the human intestine (Firmicutes: *Enterococcus faecalis, Faecalibacterium prausnitzii, Anaerostipes hadrus, Blautia coccoides, Dorea longicatena, Faecalicatena contorta* and *Ruminococcus gnavus;* Bacteroidetes: *Bacteroides fragilis, Parabacteroides distasonis, Bacteroides vulgatus* and *Bacteroides ovatus;* Actinobacteria: *Bifidobacterium longum* and *Bifidobacterium breve;* and Proteobacteria: *Escherichia coli*)*,* we observed that *M. massiliensis* was the only bacterium synthetizing high amounts of pentanoate (Figure 3A). Interestingly, gas chromatography-mass spectrometry (GC-MS) analysis revealed that, in addition to two different *M. massiliensis* strains (DSM 26228 and NCIMB 42787), *Megasphaera elsdenii,* which is the closest phylogenetic neighbor of *M. massiliensis,* also produced pentanoate, although not at as high levels as *M. massiliensis.* While most commensals generated high amounts of acetate and formate, *Faecalibacterium prausnitzii* and *Anaerostipes hadrus* synthetized high levels of butyrate. We also found that two *M. massiliensis* strains were the only producers of the medium-chain fatty acid (MCFAs) hexanoate and the branched-chain fatty acids (BCFAs) 2-methylpropionate (isobutyrate) and 3-methylbutyrate (isovalerate, isopentanoate), known to be dependent on bacterial fermentation of dietary proteins (Figure 3A). We hypothesized that bacteria producing high levels of butyrate such as *F. prausnitzii* and *A. hadrus,* or *M. elsdenii* and M. *massiliensis,* which are able to synthetize both, butyrate and pentanoate, could potentially exert HDAC inhibitory activity and may be the best candidates among commensal bacteria for adoptive CD8⁺ T cell therapy. Indeed, the supernatant of these 4 bacteria but not that of the others tested, which were not able to produce pentanoate and butyrate, strongly inhibited the activity of class I HDAC isoforms.

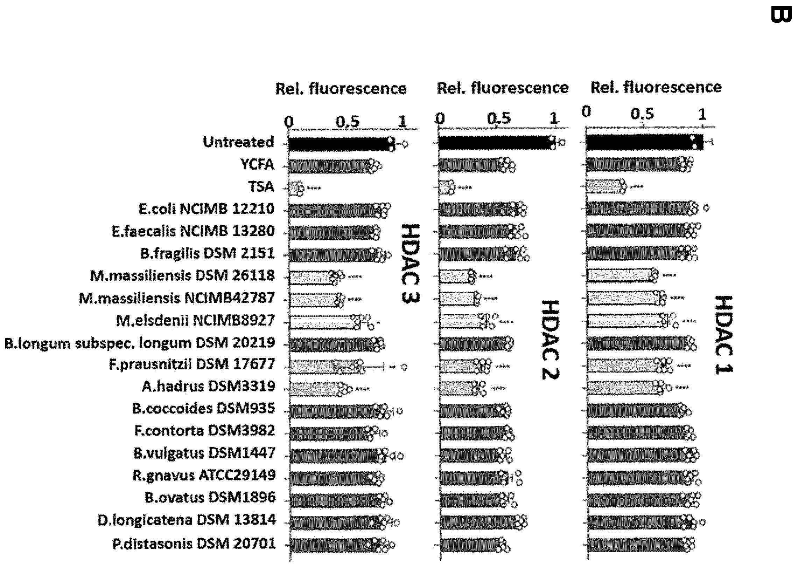

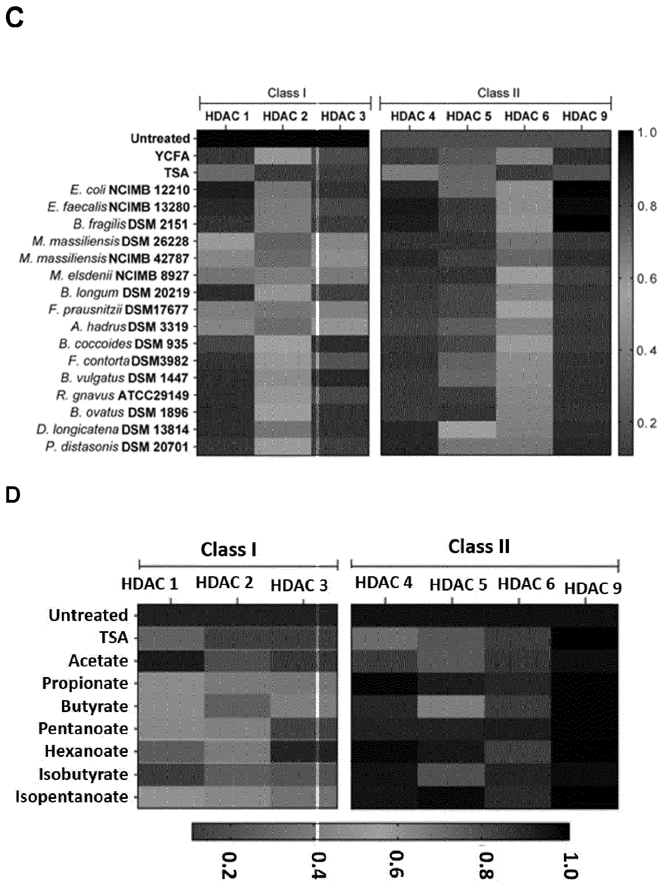

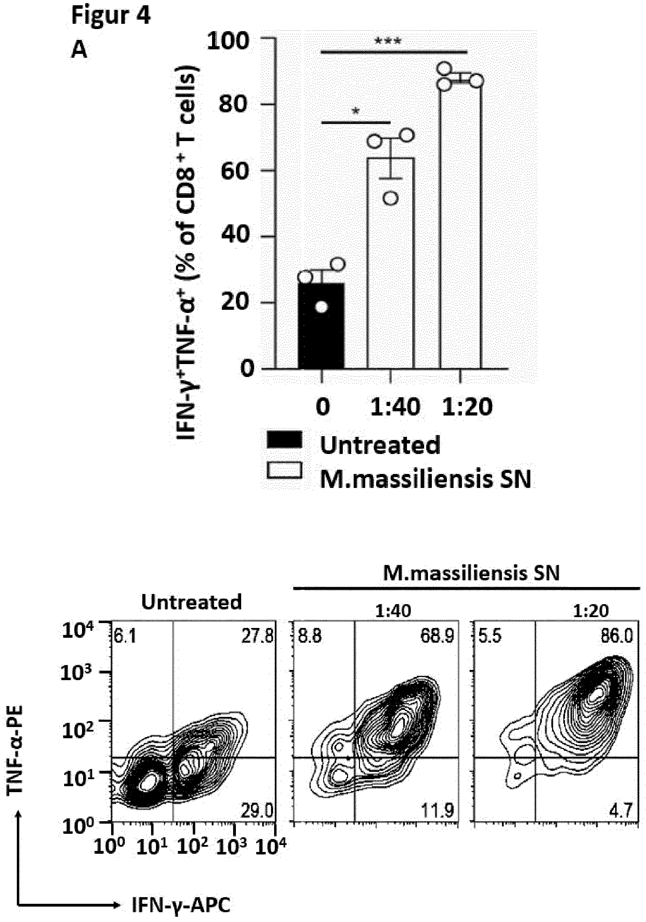

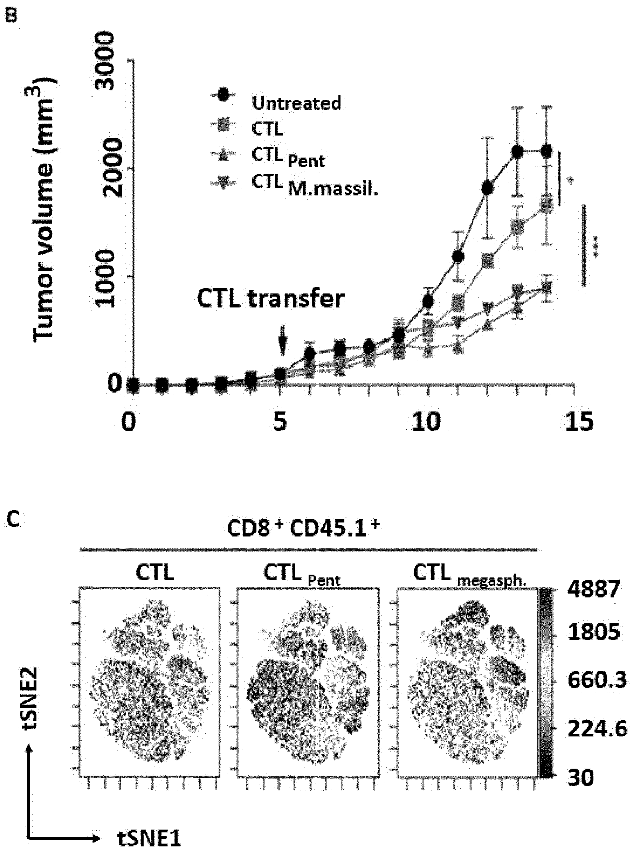

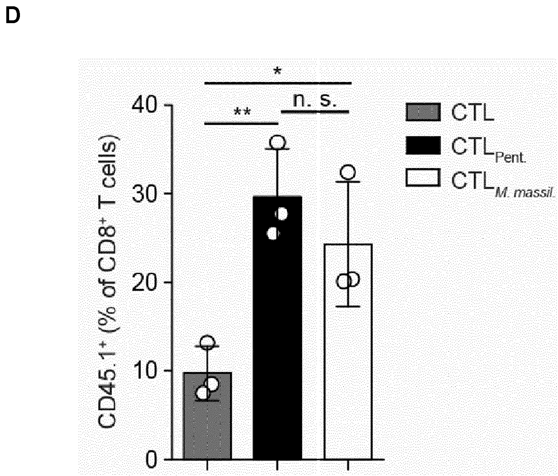

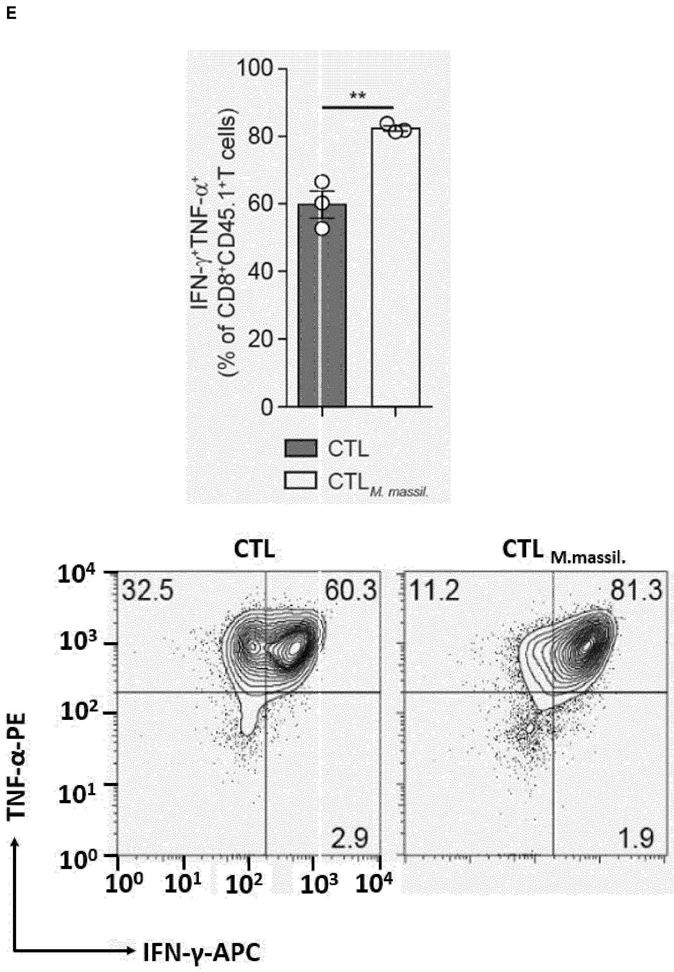

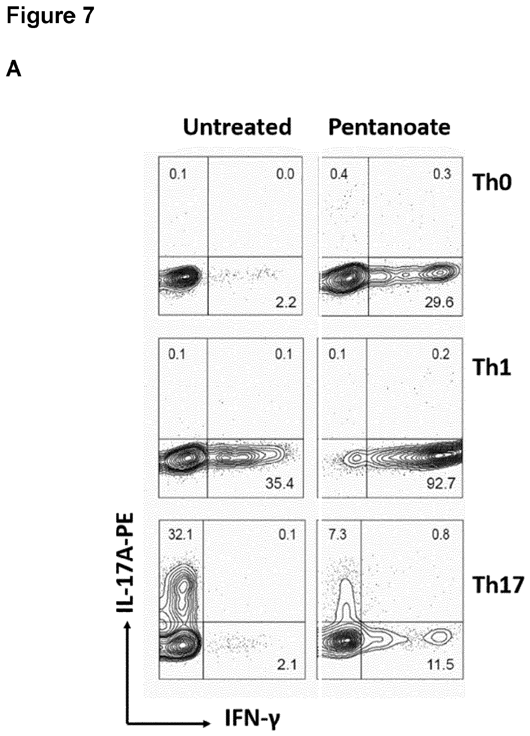

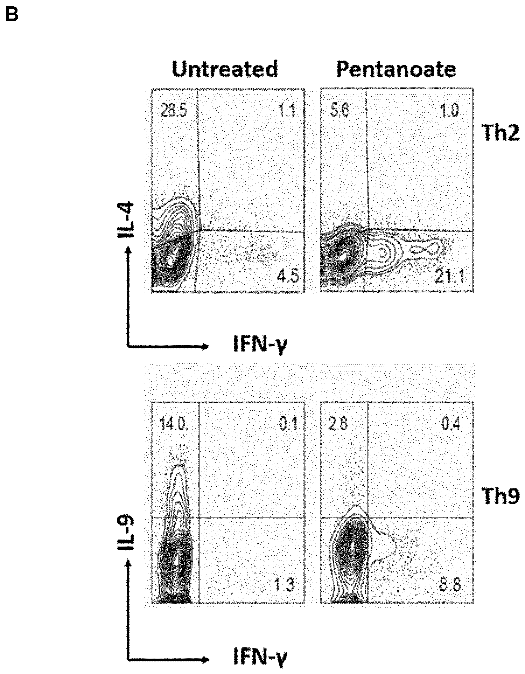

This effect was specific for class I enzymes, since class II HDACs (HDAC4, HDAC5, HDAC6 and HDAC9) were not affected by treatment with any bacterial supernatants. Particularly, the HDAC2 isoform was strongly inhibited by supernatants of *M. massiliensis,* and also by different concentrations of butyrate and pentanoate (Figure 3, B-D). The pan-HDAC inhibitor TSA inhibited both, class I and class II HDACs. When we compared the total HDAC inhibitory activity of supernatants derived from 16 human commensals, we observed that the strongest inhibitory effects were mediated by pentanoate-generating *M. massiliensis* and M. *elsdenii,* as well as by strong butyrate producers *F. prausnitzii* and *A. hadrus,* (Figure 7A). These data suggest that pentanoate- and butyrate-mediated effects on CD8⁺ T cells may be mostly mediated via inhibition of class I HDACs. We further investigated if the pentanoate-producing bacterium *M. massiliensis* has any impact on the function of CD8⁺ T cells. Indeed, the frequency of IFN-γ⁺TNF-α⁺CD8⁺ T cells and secretion of TNF-α by CTLs was markedly increased after treatment of T lymphocytes with the *M. massiliensis-derived* supernatants (Figure 4A). By comparing the supernatants derived from several gut commensal bacteria (e.g. E. *coli, E. faecalis* and B. *fragilis),* which were not able to synthetize pentanoate or butyrate, with that of *M. massiliensis,* we observed the specific effect of pentanoate-producing bacterium on TNF-α secretion (Figure 7B). Of note, Ly5.1⁺ OT-I CTLs pretreated with the supernatant of *M. massiliensis* and adoptively transferred into Ly5.2⁺ host were endowed with increased capacity to infiltrate the tumors, produce effector cytokines and eradicate B16OVA cells as compared to control CTLs (Figure 4, B-F). In summary, the broad screening revealed that only a few of the tested commensals were able to strongly inhibit HDAC activity and that strong HDAC inhibitors such as *M. massiliensis* may be used to modulate CTL function. We suggest that the identified SCFAs are novel promising candidates for supporting anti-cancer efficacy. Pentanoate seems to promote anti-tumor immunity by inhibiting class I HDAC enzymes, which results in enhancement of the acetylated state of histones at specific CTL-associated loci. Remarkably, the T cell specific deletion of class I HDAC isoforms HDAC1 and HDAC2 in CD4⁺ T cells was shown to induce strong expression of genes characteristic for CD8 lineage such as *Gzmb, Prf1* and *Eomes.* Previously, *Bifidobacterium* bacteria were shown to enhance anti-tumor immunity in mice. Interestingly, the eradication of established tumors was abrogated in CD8⁺ T cell-depleted animals, indicating that the underlying mechanism was mediated through host anti-cancer CTL responses. Similarly, *Akkermansia muciniphila* increased the recruitment of CD4⁺ T cells into tumors and restored the efficacy of ICI therapy in nonresponsive cancer patients. As *Bifidobacterium* strains and *A. muciniphila* are commensals widely present in humans, we suggest that rare gut bacterial species such as *M. massiliensis* and their specific metabolites such as pentanoate may be more adequate biotherapeutics for cancer patients.

### Pentanoate promotes the expression of CD25 and IL-2 as well as proliferative expansion and persistence of CTLs.

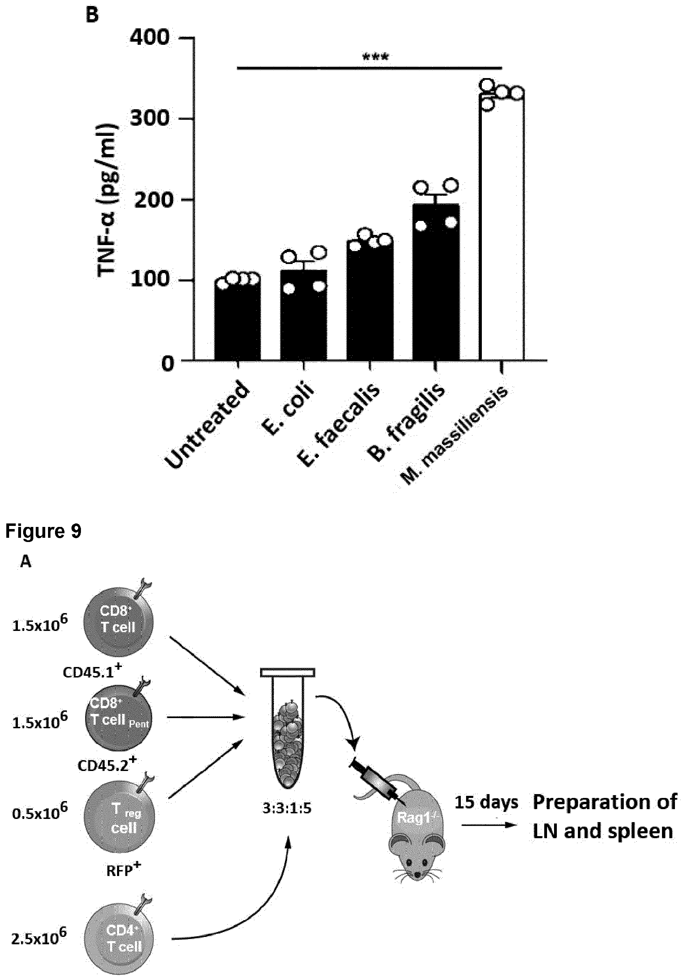

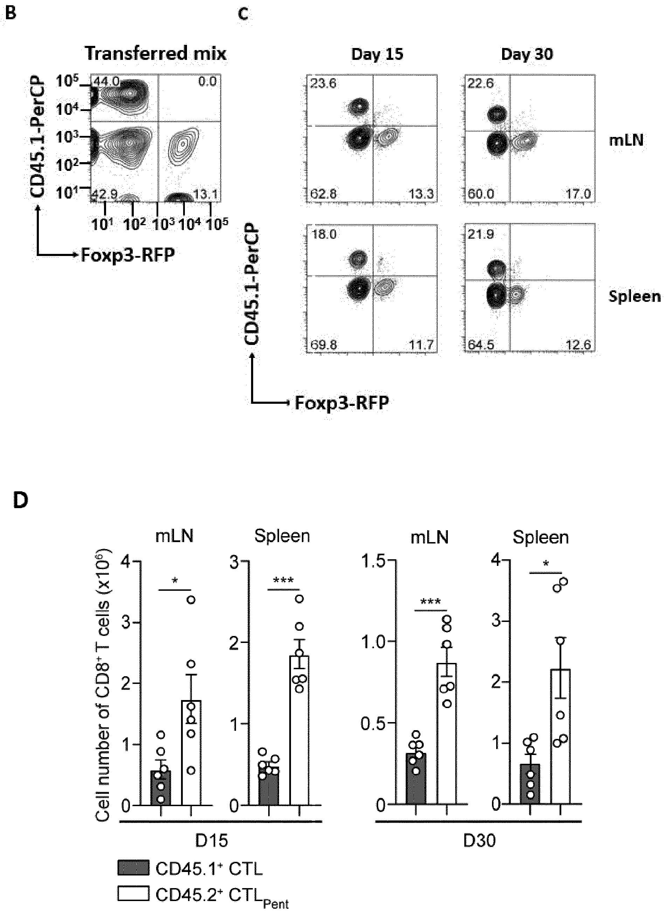

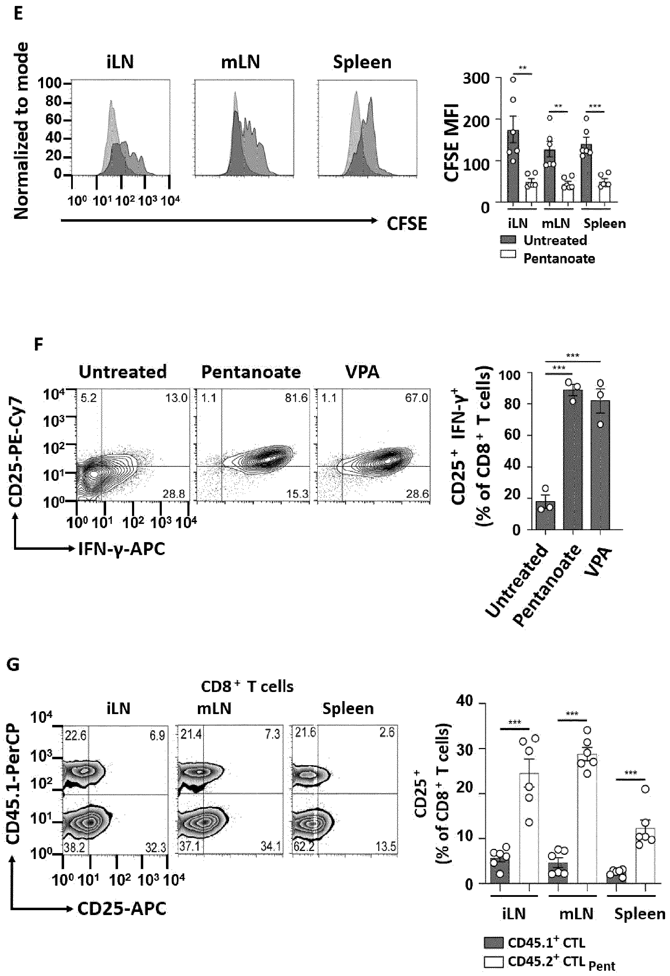

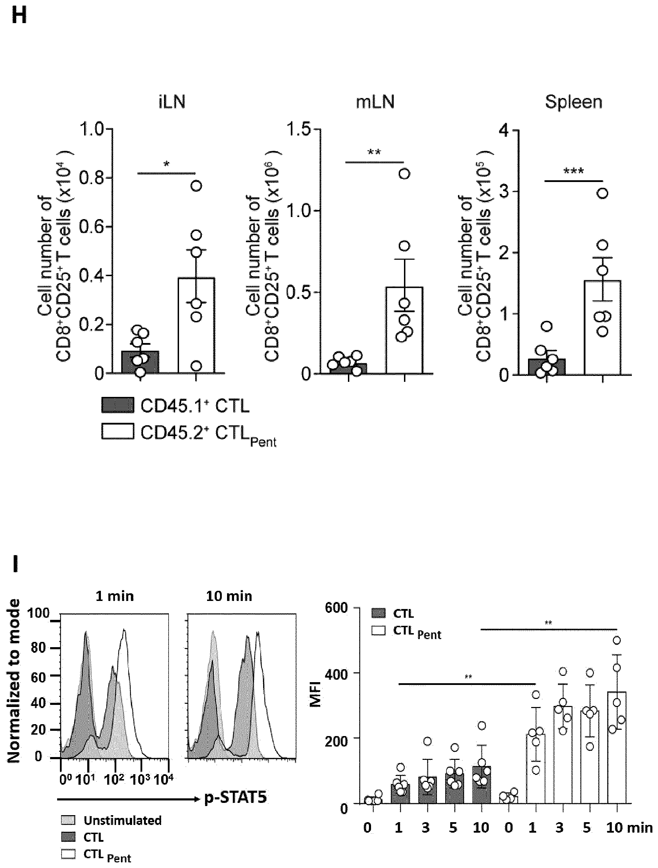

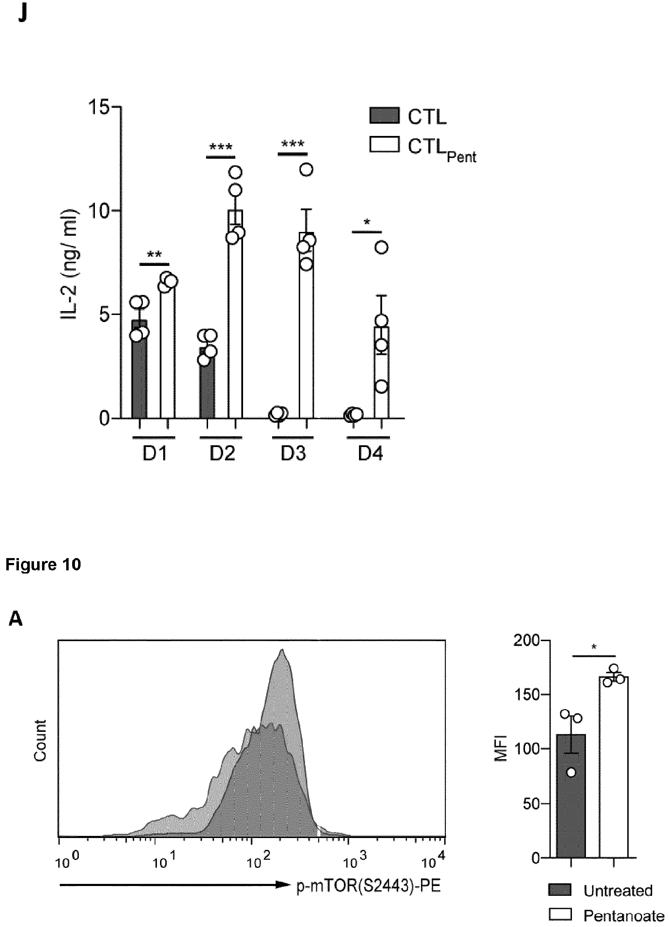

To investigate the capacity of SCFAs on survival and persistence of CD8⁺ T cells, we mixed pentanoate-treated CTLs (CD45.2⁺) with control CTLs (CD45.1⁺) at 1:1 ratio and co-transferred them into Rag1-deficient mice. To better mimic the *in vivo* tumor microenvironment, we also adoptively co-transferred *Tregs (CD45.2*⁺ *from FIR* × *tiger* reporter mice) that are frequently found in solid tumors. In addition, Foxp3⁻CD4⁺ T cells were co-transferred as the cellular source of IL-2 (Figure 9, A and B). Surprisingly, in contrast to control CTLs, pentanoate-treated CD8⁺ T lymphocytes were found at a high cell number and frequency on days 15 after transfer, even without encounter of the cognate antigen (Figure 9, C and D). The capacity of CFSE to label proliferative cells was used for in vivo monitoring of CD8⁺ T cell proliferation in Rag1-deficient mice after pretreatment with pentanoate. We found that pentanoate-treated and CFSE labelled CD8⁺ T cells had a stronger proliferative ability as compared to control CTLs (Figure 9, E). Given the importance of CD25 in supporting an effective IL-2R signalling, as well as the proliferation and survival of lymphocytes, we next asked whether pentanoate is capable of modulating CD25 expression in CTLs. Interestingly, not only pentanoate, but also valproate strongly enhanced the percentage of CD25⁺IFN-γ⁺ cells in *in vitro* generated CTLs (Figure 9, F), suggesting that the HDAC inhibitory activity of pentanoate may regulate the expression of CD25. Of note, we observed that in all three examined organs, iLN, mLN and spleen, pentanoate pre-treatment increased frequencies and numbers of CD25⁺CD8⁺ T cells as compared to control CTLs (Figure 9, G and H).

IL-2 is one of the key factors mediating proliferative expansion of T cells and among the individual receptor subunits, CD25 has the highest affinity for IL-2. By analyzing effects of pentanoate on the dynamics of IL-2-induced phosphorylation of STAT5, we found stronger STAT5 activity in response to IL-2 in pentanoate-treated CTLs in comparison to control cells (Figure 9, I). Most importantly, pentanoate-treated CTLs robustly produced IL-2 in a prolonged manner as compared to control CD8⁺ T cells (Figure 9, J), thus being able to sustain this autocrine loop for a longer period of time.

### Pentanoate modulates the cellular metabolism of CTLs by enhancing the activity of mTOR.

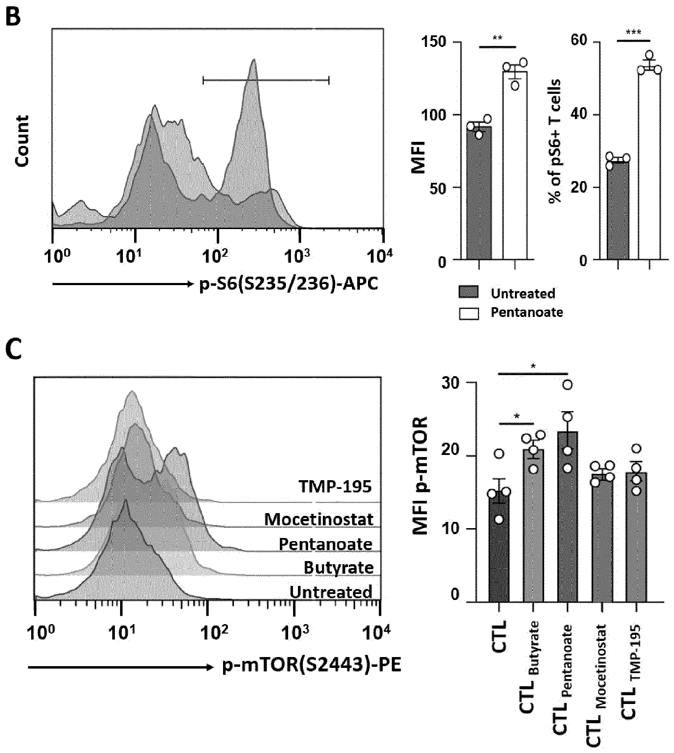

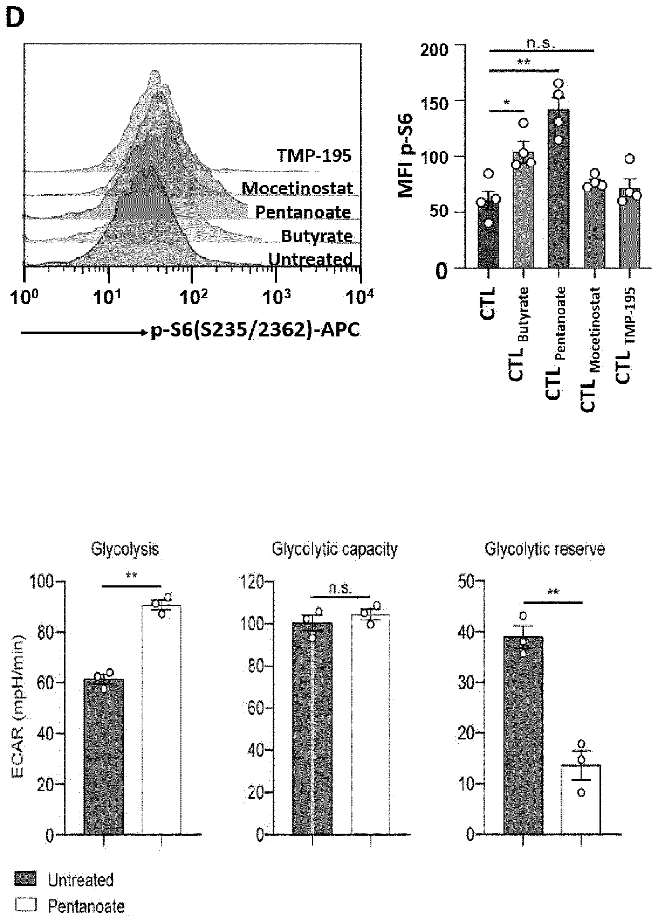

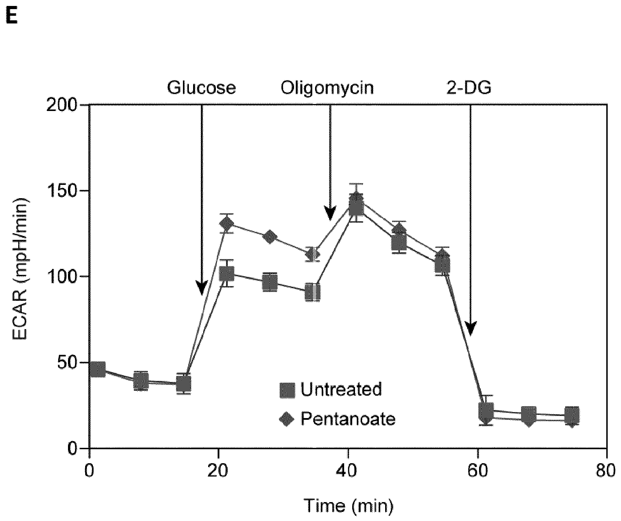

It is known that the glycolytic metabolic pathway promotes IFN-γ expression and T cell effector function. Since microbial metabolites can be utilized by T cells for metabolic demand to enhance glycolysis and oxidative phosphorylation, we tested if pentanoate is capable of increasing the activity of the mTOR complex, a key regulator of cell growth and immunometabolism. Indeed, pentanoate elevated the phosphorylation levels of both mTOR and its downstream target S6 ribosomal protein in both murine and human CTLs and CAR T cells (Figure 10, A-D). Interestingly, neither mocetinostat (HDAC class I inhibitor MGCD0103) nor TMP-195 (HDAC class II inhibitor) had a significant effect on the phosphorylation of S6, suggesting a HDAC-independent impact of pentanoate and butyrate on metabolic status of CD8⁺ T cells (Figure 10, C and D). Moreover, the extracellular acidification rate (ECAR), as an indicator of glycolytic metabolism, increased upon pentanoate treatment of CTL (Figure 10, E). Thus, similar to previously published effects of pentanoate on the metabolic activity of B cells and CD4⁺ T lymphocytes, this SCFA also enhances the glycolytic metabolism in CD8⁺ T cells.

### Pentanoate improves the efficacy of murine CAR T cells.

To gain further insight into possible therapeutic strategies, we further examined the impact of SCFAs on genetically engineered chimeric antigen receptor (CAR) T cells. For this purpose, we used CAR T cells that recognize receptor tyrosine kinase-like orphan receptor 1 (ROR1), a molecule frequently expressed in a variety of epithelial tumors and in some B cell malignancies. Previously, we could demonstrate that human CD8⁺ T lymphocytes equipped with this ROR1-CAR were able to exert potent anti-tumor effects Off note, murine ROR1-recognizing CAR T lymphocytes treated with butyrate or pentanoate enhanced their TNF-α and IFN-γ production and expression of CD25. The similar influence on CAR T cells was observed for mocetinostat, but not for TMP-195 (Figure 11, A-D). To evaluate the effect of pentanoate on murine CAR T cell *in vivo* function, we generated ROR1-expressing Panc02 pancreatic tumor cells (PancROR1) and injected them s.c. into WT mice. On day 5 after tumor cell injection, we transferred the pentanoate-treated ROR1-specific CAR T cells in tumor-bearing animals and monitored the tumor growth. By day 10 following CAR T cell administration, the tumor volume and weight were significantly diminished in mice receiving the pentanoate-treated CAR T cells as compared to animals treated with control CAR T lymphocytes (Figure 11, E) Furthermore, we found elevated frequency and number of IFN-γ ⁺TNF-α⁺ pentanoate-pretreated CAR T cells in tumors, as compared to control CAR T lymphocytes (Figure 11, F).

### Pentanoate improves the efficacy of human CAR T cells.

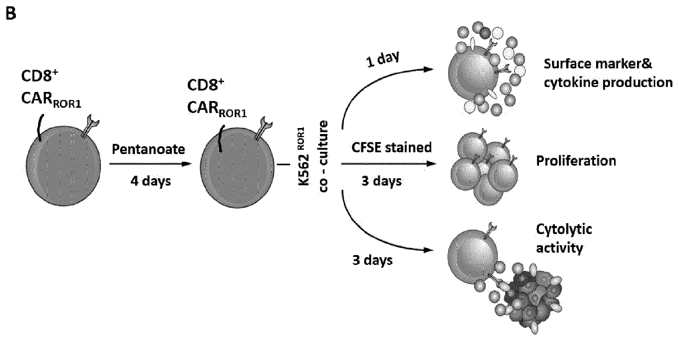

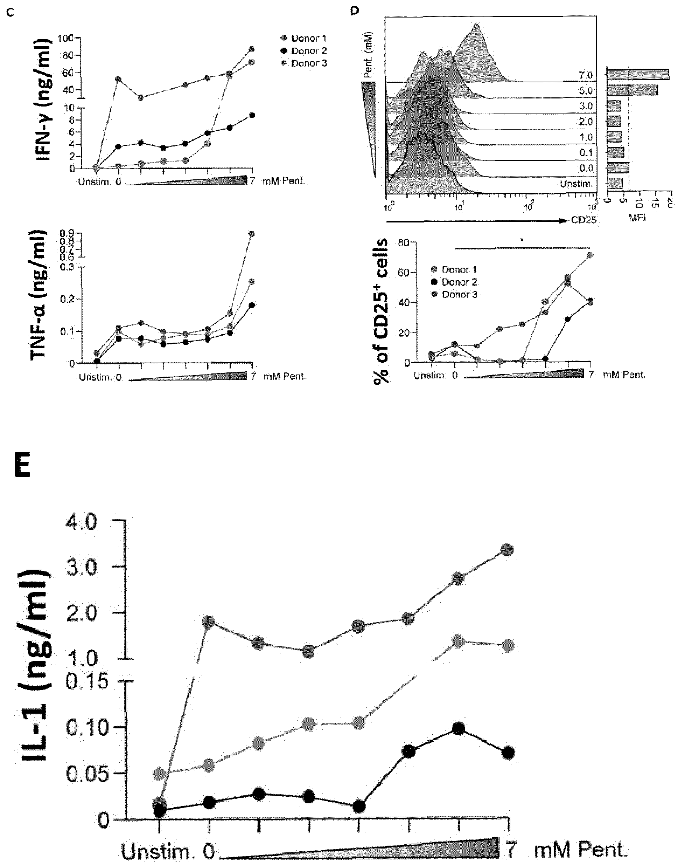

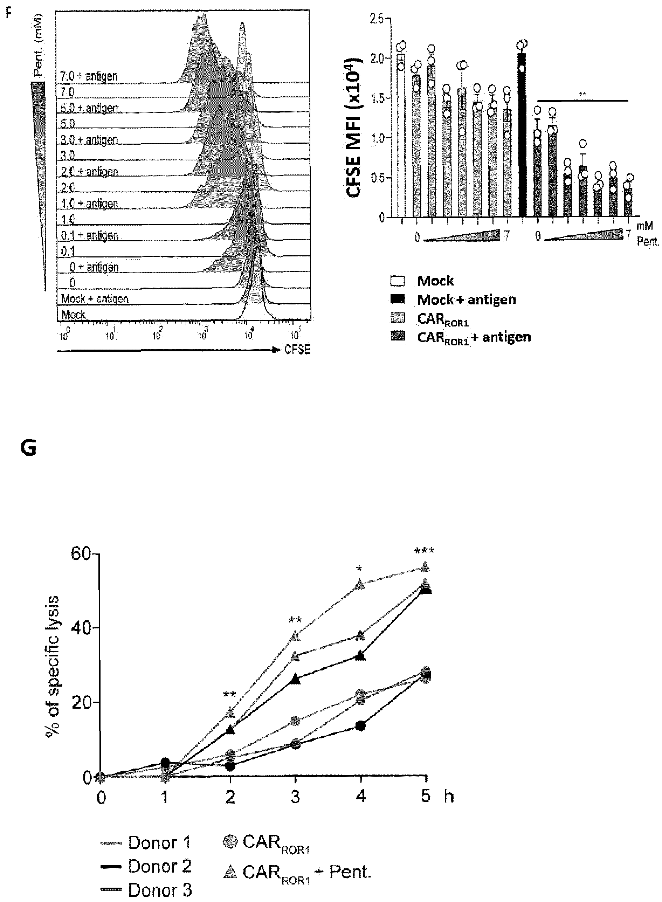

We next investigated the impact of SCFAs on human CD8⁺ T lymphocytes and CAR T cells. Our data suggest that pentanoate and butyrate could have therapeutic potential, as both SCFAs were able to induce CTL phenotype in human CD8⁺ T lymphocytes by enhancing the expression of TNF-α and IFN-γ. Again, the mocetinostat, but not the TMP-195 promoted the similar effects on human CTLs (Figure 12, A). Based on findings collected from murine CAR T cells, in a complementary approach, we pretreated human CAR T cells with pentanoate for 4 days and subsequently investigated the cytokine production, proliferation, expression of activation markers and the cytotoxic capacity of untreated and pentanoate-treated CAR T cells as indicated in the Figure 12, B. When we incubated ROR1-specific CAR T cells with ROR1-expressing K562 human cancer cells, we found that pentanoate-pretreated cells elevated the production of CTL-related cytokines IFN-γ and TNF-α (Figure 12, C). In accordance with results generated with murine CTLs, human CAR T cells also upregulated the expression of CD25 and secretion of IL-2 after pentanoate pretreatment, whereas untreated cells did not show such a strong effect (Figure 12, D and E). Moreover, we found that CAR T cells pretreated with pentanoate proliferated stronger than control CAR T cells and exerted a superior cytolytic activity after encounter of their cognate antigen (Figure 12, F and G). These results strongly suggest, that microbial metabolites such as pentanoate and butyrate may be therapeutically deployed to increase the efficacy of human CAR T cells.

### Statistics:

For all experiments, mean values of two groups were compared by using an unpaired Student's t-test (GraphPad Prism 8). P values of p < 0.05 were considered significant. Following p-values were used: *, p = 0.01-0.05; **, p = 0.001-0.01; ***, p < 0.001. Where appropriate, data are presented as means ± SEM. For comparison of multiple experimental groups, data were analyzed using the one-way analysis of variance (ANOVA).

The invention involves improving the cultivation of T cells by incubating them with short-chain fatty acid (SCFA) pentanoate after isolation from peripheral blood. The effect is that the cells are activated and the production of effector molecules is increased. This increases the chances of success of tumor therapy. This is illustrated by T-cells from mice that are transferred to mice with subcutaneous pancreatic tumors after the procedure. This type of cell treatment can be transferred to human T cells and the improved treatment of pancreatic cancer.

### Description of the Figures

**Figure 1****. Pentanoate promotes the core molecular signature of murine CD8⁺ CTLs.**
   (A) Experimental design for the treatment of CTLs with the water-soluble fraction of intestinal content derived from SPF or GF mice.
   (B and C) Frequencies of IFN-γ- and TNF-α-producing CTLs treated with the extract from luminal content derived from indicated organs. Representative results of two experiments are shown (n=3).
   (D) ELISA was performed to measure the secretion of TNF-α by CLTs treated as shown in (A).
   (E) Quantitative measurement of total SCFA amounts in fecal samples of SPF and GF mice (n =5).
   (F and G) The percentage of IFN-γ⁺TNF-α⁺ CTLs treated with the indicated SCFAs for three days (n=3, one of three independent experiments is shown).
   (H) The secretion of TNF-α from CTLs treated with SCFAs was determined by ELISA (n=3, one of two independent experiments is shown).
   (I) The fluorogenic HDAC assay was applied to measure the HDAC inhibitory activity of SCFAs on CTLs. The value for unstimulated CTLs was arbitrarily set to 1.0. Four independent experiments were performed.
   (J and K) Representative contour plots (J) and bar graphs (K) showing the frequency of IFN-γ⁺ and TNF-α⁺ cells after pentanoate (2 mM) or VPA (0.5 mM) administration. Three similar experiments were performed.
   (L and M) The frequency of *Eomes-* and T-bet-expressing CTLs treated with pentanoate (2 mM) or VPA (0.5 mM) for three days was analyzed by flow cytometry (n=4).
   (N) ChIP assay showing the acetylation of histone H4 at the promoter regions of *Ifn*γ and *Eomes* genes after 24 hours of treatment of CTLs with pentanoate. Data are from pooled chromatin of three mice.
**Figure 2****. Pentanoate enhances anti-tumor activity of antigen-specific CTLs.**
   (A) Experimental design for the role of SCFAs in promoting anti-tumor immunity.
   (B-D) After three days of pre-treatment with pentanoate, CD45.1⁺ OVA-specific CTLs were transferred intraperitoneally (i.p.) into CD45.2⁺ mice bearing 5-days old B16OVA tumors. Tumor volume and tumor mass were analyzed (n=3 mice per group, one of three independent experiments is shown).
   (E-G) The frequencies of TNF-α and IFN-γ-producing CD45.1⁺ tumor-specific CTLs in tumors, tumor-draining LNs and spleens and the absolute cell number of tumor-infiltrating CD45.1⁺CD8⁺ T cells on day 10 after adoptive transfer of cells.
   (H and I) OVA-specific CD45.1⁺ CTLs pretreated with pentanoate were adoptively transferred into CD45.2⁺ mice bearing 5-days old PancOVA tumors.
   Tumor weight was determined on day 23 post tumor inoculation. Representative data from one of two experiments are shown (n=3 mice per group).
   (J) Frequencies of transferred IFN-γ-producing CD8⁺ T cells in draining LNs and tumors on day 23 post tumor inoculation.
**Figure 3****. Bacterial-derived SCFAs exhibit specific HDAC class I inhibitory activity.**
   (A) The production of SCFAs, branched-chain fatty acids (BCFAs) and medium-chain fatty acids (MCFAs) by 16 human commensals was measured by GC-MS. All bacteria were grown *in vitro* until stationary growth phase before the measurement of fatty acids in supernatants.
   (B and C) HDAC inhibition of recombinant class I and class II HDAC isoforms by cell-free supernatants derived from 16 members of human commensal community. Significance was tested against YCFA medium.
   (D) Impact of bacterial SCFAs, BCFAs and MSCFAs on the activity of class I and II HDAC enzymes. TSA was used as a control pan-HDAC inhibitor.
**Figure 4****. Pentanoate-producing bacteria enhance CD8⁺ T cell-mediated anti-tumor immune responses.**
   (A) The frequency of IFN-γ- and TNF-α-expressing CD8⁺ T cells cultured under suboptimal CTL conditions and stimulated with supernatant derived from M. *massiliensis* (1:40 or 1:20 supernatant-to-cell media ratios). Three independent experiments were performed.
   (B-F) After three days of treatment with *M. massiliensis-derived* supernatants (1:20 supernatant-to-cell media ratio), CD45.1⁺ OT-I CTLs were transferred i.p. into CD45.2⁺ animals bearing 5-day old B16OVA tumors (n=3 mice per group, one of two similar experiments is shown). Tumor volume was analyzed at indicated time points (B).
   (C and D) The percentage of transferred CD45.1⁺ OT-I CTLs at day 14 after tumor inoculation and the illustration in the t-SNE plots are shown in (C and D). Pentanoate-treated OT-I cells served as controls.
   (E and F) The frequency and total cell numbers of transferred antigen-specific IFN-γ⁺TNF-α⁺ CTLs on day 14 after inoculation of B16OVA tumors in tumor-draining LNs are shown (E and F).
**Figure 5****. Pentanoate induces T-bet-mediated IFN-γ production via HDAC-inhibitory activity**
   (A and B) Splenic CD8⁺ T cells were polarized under suboptimal CTL-inducing conditions in presence of 1 mM pentanoate or butyrate for 3 days. Representative histogram plots (A) and bar graphs (B) are showing the frequency of Annexin V⁺ cells. Three similar experiments were performed.
   (C) Representative effector molecule staining of CD8⁺ T cells polarized under suboptimal CTL-inducing conditions, treated with pentanoate or TSA (10 nM Sigma-Aldrich) in presence of 2-DG (1 mM, Sigma-Aldrich) or rapamycin (100 nM, Sigma-Aldrich) for 3 days. Three independent experiments were performed.
   (D) Western blot analysis of H3Ac and H4Ac in CD8⁺ T cells treated with SCFAs for 3 days.
   (E) Pan-H4Ac ChIP qPCR analysis for *Tbx21* promoter accessibility in pentanoate-treated CTLs. Data are from pooled chromatin of three mice.
   (F and G) CD8⁺ T cells isolated from WT and *Tbx21*^{-/-} mice were polarized under suboptimal CTL-inducing conditions in presence of increasing pentanoate concentrations. Representative contour plots (F) and bar graphs (G) show the frequency of IFN-γ⁺ and IL-17A⁺ cells. Two similar experiments were performed.
**Figure 6****. Pentanoate induces CTL phenotype in Tc17 and Tc9 cells**
   (A and B) CD8⁺ T cells isolated from spleens and LNs were polarized under Tc17-inducing conditions and treated with pentanoate for 3 days. Representative contour plots (A) and bar graphs (B) indicate the frequency of IL-17⁺ and IFN-γ⁺ cells. Three similar experiments were performed.
   (C and D) CD8⁺ T cells isolated from spleens and LNs were polarized under Tc9-inducing conditions and treated with pentanoate for 3 days. Representative contour plots (C) and bar graphs (D) are showing the frequency of IL-9⁺ and IFN-γ⁺ cells. Three similar experiments were performed.
   (E and F) The expression of granzyme B, IFN-γ and *Eomes* in pentanoate-treated human CTLs was analysed by flow cytometry. Five independent experiments were performed (E). For *Eomes,* mean fluorescence intensity (MFI) values are shown (F, n=3).

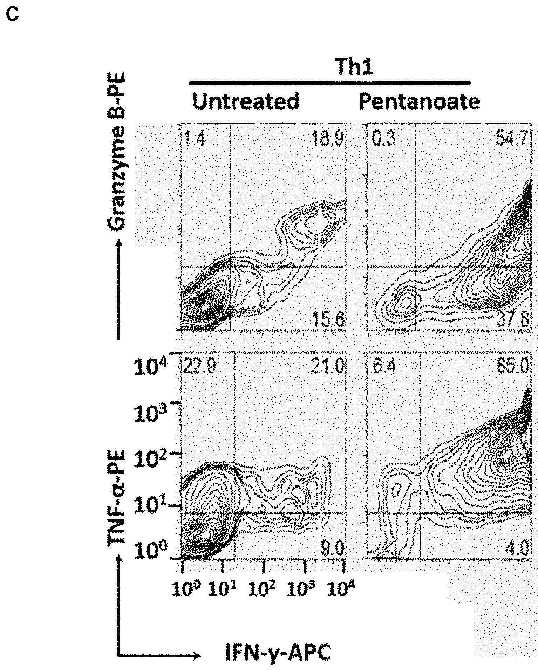

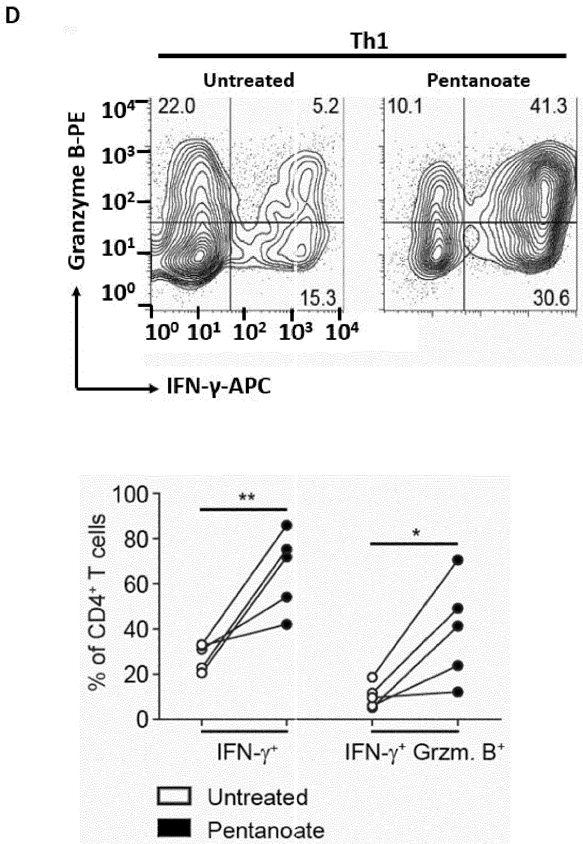

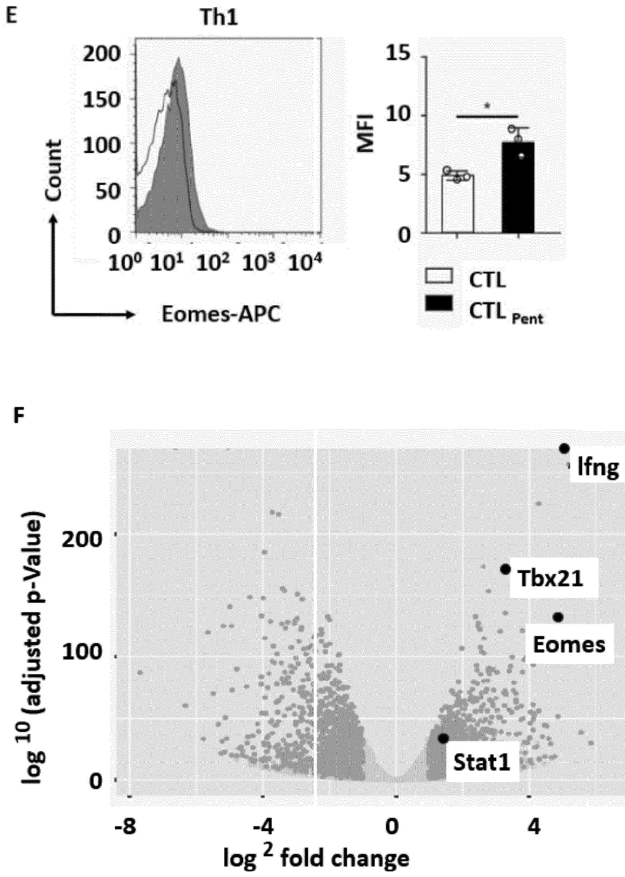

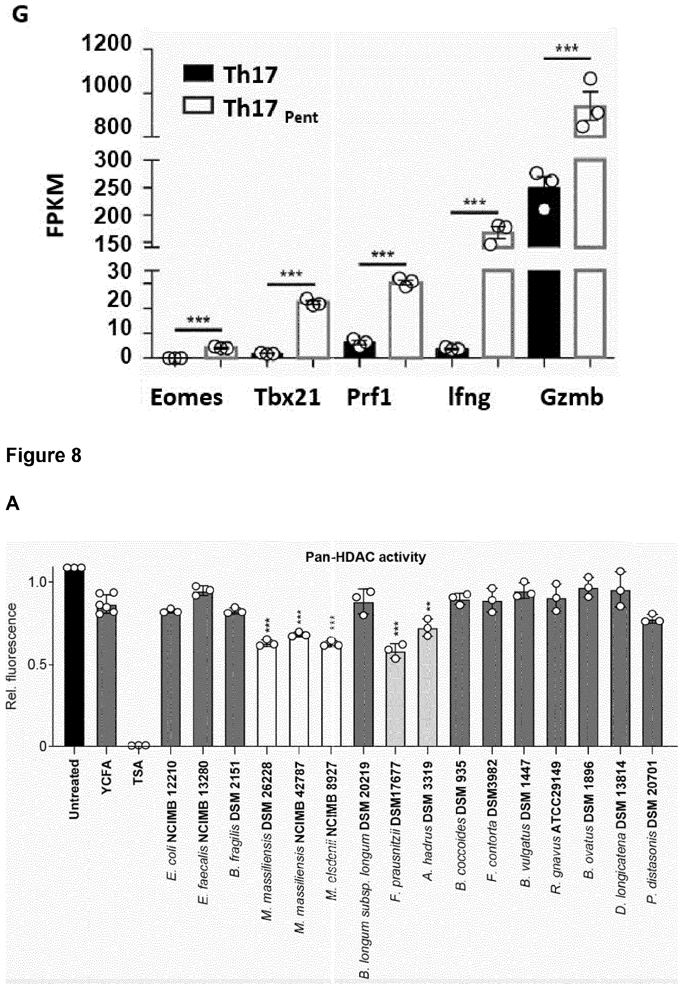

**Figure 7****. Pentanoate induces CTL phenotype in CD4⁺ T cell subsets**
   (A and B) CD4⁺ T cells isolated from spleens and LNs were treated with pentanoate (2 mM) for 3 days under Th-polarizing conditions and stained for production of their signature cytokines. Three similar experiments were performed. (C) Pentanoate-treated CD4⁺ T cells under Th1-polarizing conditions were stained for CTL effector molecules. A representative contour blot of three independent experiments is shown.
   (D and E) The expression of granzyme B, IFN-γ and *Eomes* in pentanoate-treated human CTLs was analyzed by flow cytometry. Five experiments were performed (D). For *Eomes,* mean fluorescence intensity (MFI) values are shown (E, n=3).
   (F) RNA-seq analysis of CD4⁺ Th17 cells in the presence of pentanoate. Volcano plot with differentially regulated genes is shown.
   (G) Expression of CTL-associated genes in pentanoate-treated Th17 cells. Results of RNA-seq analysis for indicated genes are displayed as reads per kilobase per million mapped reads (RPKM).
**Figure 8****. Impact of cell-free supernatants of various human commensals on HDAC enzymes.**
   (A) Screening for total HDAC inhibitory effects of cell-free supernatants derived from 16 bacterial strains on cell lysates derived from human HT-29 cells (B). Significances were tested against YCFA medium.
   (B) The secretion of TNF-α from CTLs was measured by ELISA after three days of stimulation with supernatants of indicated bacteria (n=4, 1:20 supernatant-to-cell media ratio for all bacteria).
**Figure 9****. Pentanoate promotes the expression of CD25 and IL-2 as well as proliferative expansion and persistence of CTLs.**
   (A) experimental setup for investigating CTL persistence *in vivo* is shown.
   (B-D) The frequency (B, C) and total cell numbers (D) of transferred T cells (WT CD45.1⁺ CTLs, WT CD45.2⁺ pentanoate-treated CTLs and Foxp3⁺CD45.2⁺ Tregs from *FIR* × *tiger* mice) in Rag1-deficient mice on days 15 after the adoptive transfer are shown. The co-transferred Foxp3⁻CD4⁺ cells were excluded from the gate (B, C). In D, n = 3 mice/group/experiment, data from 2 pooled independent experiments are shown.
   (E) The CFSE label of transferred T cells (WT CD45.1⁺ CTLs, WT CD45.2⁺ pentanoate-treated CTLs and CD45.2⁺ CD4⁺ T cells) in Rag1-deficient mice on days 4 after the adoptive transfer are shown ( n = 3 mice/group/experiment, data pooled from 2 independent experiments are shown).
   (F) The percentage of CD25⁺IFN-□⁺CTLs treated with indicated HDAC inhibitors for three days (n = 3, performed in 3 independent experiments).
   (G and H) Frequencies and cell numbers of CD25⁺CD8⁺ T cells were analysed by flow cytometry on day 15 after transfer of control CTLs (CD45.1⁺) and pentanoate-treated CTLs (CD45.2⁺) into Rag1-deficient mice (n = 3 mice/group/experiment, data pooled from 2 independent experiments).
   (I) CTLs were generated in the presence or absence of pentanoate for 3 day, then washed and rested for 4 hours. Subsequently, cells were treated with IL-2 (50 U/ml) for indicated time points. The phosphorylated (p)-STAT5 levels were analysed by flow cytometry (n = 5, performed in 3 independent experiments).
   (J) The secretion of IL-2 in pentanoate-treated CTLs cultured for indicated time points was measured by ELISA (n = 4, pooled from 2 independent experiments).
**Figure 10****. Pentanoate induces the mTOR pathway in CTLs.**
   (A and B) CTLs were cultured in medium containing 1.0% FCS and treated with pentanoate (2.5 mM) for three days. Representative histogram plots and bar graphs indicate the phoshorylated levels of mTOR (A) and S6 ribosomal protein (B), respectively (n = 3 pooled from three independent experiments).
   (C and D) Human CTLs were cultured in medium containing 1.0% serum and treated with indicated HDACi for three days. Representative histogram plots and bar graphs indicate the phoshorylated levels of mTOR (C) and S6 ribosomal protein (D), respectively (data points represent four individual healthy donors)
   (E) Measurement of extracellular acidification rate (ECAR) for *in vitro* generated murine CTLs cultured with or without 2.5 mM pentanoate for three days. ECAR was measured under basal conditions and in response to glucose (10 mM), oligomycin (2 µM), and 2-deoxy-glucose (2-DG, 100 mM). One of three independent experiments is shown.
**Figure 11****. Pentanoate-treatment enhances the anti-tumor activity of murine CAR T cells.**
   (A) Experimental design for the analysis HDACi-treated ROR1-specific CD8⁺ CAR T cells (CAR_{ROR1}+HDACi).
   (B and C) The production of TNF-α and IFN-γ from CAR T cells was measured by ELISA and flow cytometry analysis after three days of stimulation with the indicated HDACi.
   (D) The surface expression of CD25 on CAR T cells was measured by flow cytometry analysis after three days of stimulation with the indicated HDACi (n = 3 combined from 3 independent experiments).
   (E and F) After three days of pre-treatment with pentanoate, CD19t⁺ ROR1-specific CAR T cells were transferred intraperitoneally (i.p.) into mice bearing 5-days old PancROR1 tumors. In E, Tumor volume and tumor mass were analyzed (n = 3 mice/group/experiment combined from 2 independent experiments).
   (F) The percentage and total cell number of transferred TNF-α and IFN-γ CD19t⁺ ROR1-specific CAR T cells in the tumor tissue at day 14 after tumor inoculation are shown.
**Figure 12****. Pentanoate enhances the functional status of human CAR T cells.**
   (A) CD8⁺ T cells isolated from peripheral blood of healthy donors were differentiated into CTLs in presence or absence of indicated HDACi. Representative contour plots and dot plots indicate the frequency of TNF-α + and IFN-γ ⁺ cells. Data points in the graphs represent individual donors (n = 4, performed in 4 independent experiments)
   (B-G) Phenotyping of pentanoate-treated ROR1-specific CD8⁺ CAR T cells (CAR_{ROR1} T cells).
   (B) Experimental setup for the functional analysis is shown.
   (C) The cytokine secretion (IFN-γ and TNF-α) was analysed in supernatants of pentanoate-treated CAR_{ROR1} T cells by ELISA.
   (D) The surface expression of CD25 was measured by flow cytometry.
   (E) The secretion of IL-2 was detected in supernatants of CAR_{ROR1} T cells by ELISA.
   (F) Proliferation of CAR_{ROR1} T cells was determined by CFSE labelling. CAR_{ROR1} T cells pre-treated with pentanoate were stained with CFSE and subsequently co-cultured with K652^{ROR1} cells in the absence of pentanoate. CD8⁺ T cells without the CAR construct were used as mock control cells.
   (G) The cytolytic activity of CAR_{ROR1} T cells was examined by analysis of specific lysis following encounter with luciferase-expressing K652^{ROR1} cells. The percentage of lysed target cells was determined in 1 hour intervals (effector-to-target cell (E:T) ratio = 2.5:1). Data points shown in the graphs (C-G) represent CAR_{ROR1} T cells derived from three different donors. Following pentanoate pre-treatment, the stimulation was mediated by co-culture of CD8⁺ CAR_{ROR1} T cells with ROR1-expressing K652 (K652^{ROR1}) cells in the absence of pentanoate.

## Claims

1. Method for the activation of immune cells by incubating them with at least one short-chain fatty acid so that the immune cells are activated so that they can increase the production of effector molecules,
wherein the immune cells are chimeric antigen receptor (CAR) T cells,
and wherein the activation of the immune cells by short-chain fatty acids is performed *in vitro.*

2. Method according to claim 1 **characterized in that** the short-chain fatty acid comprises pentanoate or a pharmaceutical acceptable derivative thereof.

3. Method according to claim 1 to 2 **characterized in that** the short-chain fatty acid comprises pentanoate and butyrate or pharmaceutical acceptable compositions thereof.

4. Method according to claim 1 to 3 **characterized in that** the at least one short-chain fatty acid is produced by at least one species of bacteria.

5. Method according to claim 4 **characterized in that** the at least one short-chain fatty acid is produced by the bacterium *Megasphaera massiliensis.*

6. Method according to claim 4 and 5 **characterized in that** the at least one short-chain fatty acid is produced by a group of bacteria comprising at least the bacteria *Megasphaera massiliensis, Megasphaera elsdenii, Faecalibacterium prausnitzii* and *Anaerostipes hadrus.*

## Patentansprüche

1. Verfahren zur Aktivierung von Immunzellen durch Inkubation mit mindestens einer kurzkettigen Fettsäure, so dass die Immunzellen aktiviert werden und vermehrt Effektormoleküle produzieren können,
wobei die Immunzellen chimärische Antigenrezeptor-T-Zellen (CAR) sind,
und wobei die Aktivierung der Immunzellen durch kurzkettige Fettsäuren *in vitro* durchgeführt wird.

2. Verfahren nach Anspruch 1, **dadurch gekennzeichnet, dass** die kurzkettige Fettsäure Pentanoat oder ein pharmazeutisch akzeptables Derivat davon umfasst.

3. Verfahren nach Anspruch 1 bis 2, **dadurch gekennzeichnet, dass** die kurzkettige Fettsäure Pentanoat und Butyrat oder pharmazeutisch akzeptable Zusammensetzungen davon umfasst.

4. Verfahren nach Anspruch 1 bis 3, **dadurch gekennzeichnet, dass** die mindestens eine kurzkettige Fettsäure von mindestens einer Bakterienart produziert wird.

5. Verfahren nach Anspruch 4, **dadurch gekennzeichnet, dass** die mindestens eine kurzkettige Fettsäure von dem Bakterium *Megasphaera massiliensis* produziert wird.

6. Verfahren nach Anspruch 4 und 5, **dadurch gekennzeichnet, dass** die mindestens eine kurzkettige Fettsäure von einer Gruppe von Bakterien produziert wird, zu der mindestens die Bakterien *Megasphaera massiliensis, Megasphaera elsdenii, Faecalibacterium prausnitzii* und *Anaerostipes hadrus* gehören.

## Revendications

1. Procédé d'activation des cellules immunitaires par leur incubation avec au moins un acide gras à chaîne courte de sorte que les cellules immunitaires sont activées de manière à pouvoir augmenter la production de molécules effectrices, dans lequel les cellules immunitaires sont des lymphocytes T à récepteurs antigéniques chimériques (CAR), et dans lequel l'activation des cellules immunitaires par des acides gras à chaîne courte est réalisée *in vitro.*

2. Procédé selon la revendication 1, **caractérisé en ce que** l'acide gras à chaîne courte comprend du pentanoate ou un dérivé pharmaceutiquement acceptable de celui-ci.

3. Procédé selon les revendications 1 à 2 **caractérisé en ce que** l'acide gras à chaîne courte comprend du pentanoate et du butyrate ou des compositions pharmaceutiquement acceptables de ceux-ci.

4. Procédé selon la revendication 1 à 3 **caractérisé en ce que** l'au moins un acide gras à chaîne courte est produit par au moins une espèce de bactéries.

5. Procédé selon la revendication 4 **caractérisé en ce que** l'au moins un acide gras à chaîne courte est produit par la bactérie *Megasphaera massiliensis.*

6. Procédé selon la revendication 4 et la revendication 5 **caractérisé en ce que** l'au moins un acide gras à chaîne courte est produit par un groupe de bactéries comprenant au moins les bactéries *Megasphaera massiliensis, Megasphaera elsdenii, Faecalibacterium prausnitzii* et *Anaerostipes hadrus.*
